# EUROPÄISCHE PATENTSCHRIFT

(11) **EP 2 699 641 B1**
(45) Veröffentlichungstag und Bekanntmachung des Hinweises auf die Patenterteilung: **17.05.2017**
(21) Anmeldenummer: 12710148.3
(22) Anmeldetag: 23.03.2012
(51) Int. Cl.: C09K 11/06, H05B 33/14, H01L 51/50, H01L 51/00

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 18.04.2011 EP 11003230
(43) Veröffentlichungstag der Anmeldung: 26.02.2014
(73) Patentinhaber: Merck Patent GmbH, 64293 Darmstadt (DE)
(72) Erfinder: STOESSEL, Philipp, 60487 Frankfurt am Main (DE); BUESING, Arne, 65929 Frankfurt am Main (DE); VOGES, Frank, 67098 Bad Duerkheim (DE); PFLUMM, Christof, 60316 Frankfurt am Main (DE); PARHAM, Amir Hossain, 65929 Frankfurt am Main (DE); EBERLE, Thomas, 76829 Landau (DE); MUJICA-FERNAUD, Teresa, 64283 Darmstadt (DE)
(86) Internationale Anmeldenummer: PCT/EP2012/001285
(87) Internationale Veröffentlichungsnummer: WO 2012/143079

(56) Entgegenhaltungen:
- WO-A1-2008/086851
- JP-A- 2006 080 271
- US-A- 5 508 136
- US-A1- 2002 045 061

## Beschreibung

Gegenstand der vorliegenden Erfindung ist eine Verbindung einer Formel (I), die Verwendung dieser Verbindung in einer elektronischen Vorrichtung, sowie eine elektronische Vorrichtung, enthaltend eine oder mehrere Verbindungen der Formel (I). Weiterhin betrifft die Erfindung die Herstellung der Verbindung der Formel (I) sowie eine Formulierung enthaltend eine oder mehrere Verbindungen der Formel (I).

Die Entwicklung von neuartigen funktionellen Verbindungen zur Verwendung in elektronischen Vorrichtungen ist aktuell Gegenstand intensiver Forschung. Ziel ist hierbei die Entwicklung und Untersuchung von Verbindungen, welche bislang noch nicht in elektronischen Vorrichtungen eingesetzt wurden, sowie die Entwicklung von Verbindungen, welche ein verbessertes Eigenschaftsprofil der Vorrichtungen ermöglichen.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Der Aufbau organischer Elektrolumineszenzvorrichtungen (OLEDs), in denen die Verbindungen gemäß Formel (I) bevorzugt ais funktionelle Materialien eingesetzt werden können, ist dem Fachmann bekannt und unter anderem in US 4539507, US 5151629, EP 0676461 und WO 1998/27136 beschrieben.

Betreffend die Leistungsdaten der organischen Elektrolumineszenzvorrichtungen sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der organischen Elektrolumineszenzvorrichtungen sowie die realisierten Farbwerte. Insbesondere bei blau emittierenden Elektrolumineszenzvorrichtungen besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht Bedarf an alternativen Lochtransportmaterialien zur Verwendung in elektronischen Vorrichtungen. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Gemäß dem Stand der Technik werden als Lochtransportmaterialien in der Lochtransportschicht bzw. in der Lochinjektionsschicht insbesondere Triarylaminderivate verwendet, welche zwei Triarylaminogruppen aufweisen. Diese Verbindungen leiten sich häufig von Diarylamino-substituierten Triphenylaminen (TPA-Typ), von Diarylamino-substituierten Biphenyl-Derivaten (TAD-Typ) oder Kombinationen dieser Grundverbindungen ab. Weiterhin werden beispielsweise Spirobifluorenderivate eingesetzt, welche mit zwei oder vier Diarylaminogruppen substituiert sind (z. B. gemäß EP 676461). Bei diesen Verbindungen gibt es sowohl bei fluoreszierenden wie auch bei phosphoreszierenden OLEDs weiterhin Verbesserungsbedarf, insbesondere hinsichtlich Effizienz, Lebensdauer und Betriebsspannung bei Verwendung in einer organischen Elektrolumineszenzvorrichtung sowie hinsichtlich der thermischen Stabilität bei der Sublimation.

Weiterhin sind im Stand der Technik sogenannte Starburst-Amine zur Verwendung in OLEDs bekannt. Starburst-Amine, welche drei Diarylaminogruppen aufweisen, die an einen gemeinsamen zentralen Benzolring gebunden sind, wobei der zentrale Benzolring an den verbleibenden Positionen unsubstituiert ist, sind unter anderem in EP 0611148 zur bevorzugten Verwendung in einer Lochtransportschicht offenbart. Zur Verwendung als lochtransportierendes Material in einer emittierenden Schicht enthaltend blau oder grün phosphoreszierende Emitterverbindungen sind diese Verbindungen jedoch nur in geringem Maße geeignet.

Es besteht weiterhin Bedarf an alternativen Matrixmaterialien zur Verwendung in elektronischen Vorrichtungen. Insbesondere besteht Bedarf an Matrixmaterialien für phosphoreszierende Emitter, die gleichzeitig zu guter Effizienz, hoher Lebensdauer und geringer Betriebsspannung führen. Gerade die Eigenschaften der Matrixmaterialien sind häufig limitierend für die Lebensdauer und die Effizienz der organischen Elektrolumineszenzvorrichtung. Insbesondere ist es für Matrixmaterialien für phosphoreszierende Emitter wünschenswert, dass diese ein hochliegendes T₁-Niveau (Triplettniveau) aufweisen.

Gemäß dem Stand der Technik werden häufig Carbazolderivate, z. B. Bis(carbazolyl)biphenyl, als Matrixmaterialien verwendet. Weiterhin werden Ketone (WO 2004/093207), Phosphinoxide, Sulfone (WO 2005/003253), Triazinverbindungen wie Triazinylspirobifluoren (vgl. WO 2005/053055 und WO 2010/015306) sowie Metallkomplexe, beispielsweise BAlq oder Bis[2-(2-benzothiazol)phenolat]-zink(II), als Matrixmaterialien für phosphoreszierende Emitter verwendet. Es besteht jedoch weiterhin Bedarf an alternativen Verbindungen zur Verwendung als Matrixmaterialien für phosphoreszierende Emitter. Insbesondere besteht Bedarf an Verbindungen, mit denen eine Verbesserung der Leistungsdaten der elektronischen Vorrichtungen erzielt werden kann.

JP 2006-80271 beschreibt Verbindungen, die einen Benzolkern aufweisen können und mit Carbazolgruppen substituiert sind für die Anwendung in organischen Elektrolumineszenzvorrichtungen.

WO2008/086851 beschreibt Verbindungen, die einen Benzolkern aufweisen können und mit Carbazolgruppen substituiert sind für die Anwendung in organischen Elektrolumineszenzvorrichtungen.

US 2002/0045061 beschreibt Verbindungen, die einen Benzolkern aufweisen können und mit Carbazolgruppen substituiert sind für die Anwendung in organischen Elektrolumineszenzvorrichtungen.

Der vorliegenden Erfindung liegt somit die technische Aufgabe zu Grunde, Verbindungen bereitzustellen, welche sich zur Verwendung in elektronischen Vorrichtungen wie beispielsweise OLEDs eignen, und welche insbesondere als Lochtransportmaterialien und als Matrixmaterialien für phosphoreszierende Emitter eingesetzt werden können.

Im Rahmen der vorliegenden Erfindung wurde nun gefunden, dass sich Verbindungen wie in Anspruch 1 definiert ausgezeichnet zur Verwendung in elektronischen Vorrichtungen, insbesondere als Lochtransportmaterialien und als Matrixmaterialien für phosphoreszierende Emitter, eignen. Diese Verbindungen besitzen kennzeichnenderweise drei Arylaminogruppen bzw. N-heterocyclische Gruppen, welche an einen gemeinsamen zentralen Benzolring gebunden sind. Weiterhin sind die Verbindungen dadurch gekennzeichnet, dass sie an den verbleibenden drei Positionen des zentralen Benzolrings substituiert sind.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenden Heteroatome, so gelten diese.

Unter einer Arylgruppe bzw. Heteroarylgruppe wird im Rahmen der vorliegenden Erfindung entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Eine Aralkylgruppe im Sinne dieser Erfindung ist eine mit einer Arylgruppe substituierte Alkylgruppe, wobei der Begriff Arylgruppe wie oben definiert zu verstehen ist und die Alkylgruppe 1 bis 20 C-Atome aufweist, wobei in der Alkylgruppe auch einzelne H-Atome und/oder CH₂-Gruppen durch die bei der Definition der Alkylgruppen genannten Gruppen ersetzt sein können und wobei die Alkylgruppe diejenige Gruppe darstellt, welche an den Rest der Verbindung bindet. Entsprechend stellt eine Heteroaralkylgruppe eine mit einer Heteroarylgruppe substituierte Alkylgruppe dar, wobei der Begriff Heteroarylgruppe wie oben definiert zu verstehen ist und die Alkylgruppe 1 bis 20 C-Atome aufweist, wobei in der Alkylgruppe auch einzelne H-Atome und/oder CH₂-Gruppen durch die bei der Definition der Alkylgruppen genannten Gruppen ersetzt sein können und wobei die Alkylgruppe diejenige Gruppe darstellt, welche an den Rest der Verbindung bindet.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, **O** und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³- hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluor-ethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

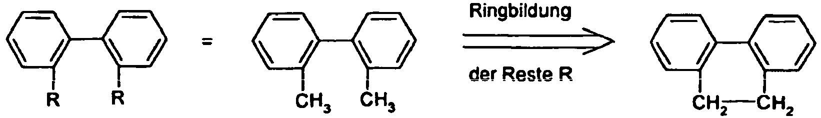

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies wird durch das folgende Schema verdeutlicht:

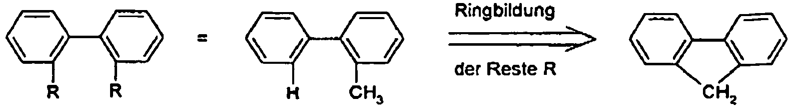

Gemäß der vorliegenden Erfindung kann die Verbindung der Formel (I) den folgenden Formeln (I-1) oder (I-2) entsprechen: 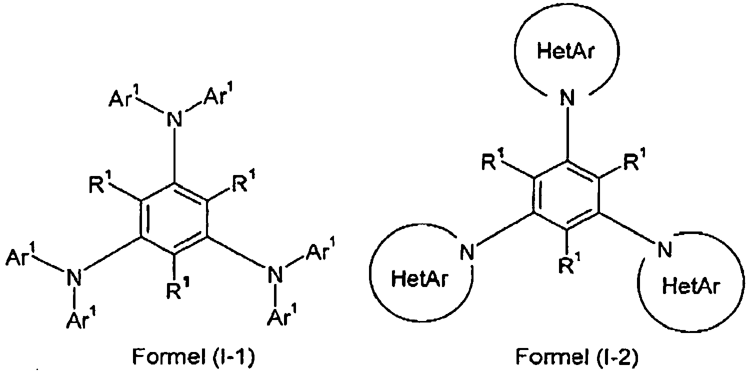 wobei die Symbole definiert sind wie oben im Zusammenhang mit der Definition der Formel (I).

Ar¹ als Bestandteil der Gruppe A der Formel (II) stellt eine Arylgruppe mit 6 bis 10 aromatischen Ringatomen dar, bevorzugt eine Arylgruppe mit 6 aromatischen

Ringatomen, wobei Ar¹ mit einem oder mehreren Resten R² substituiert sein kann.

Gemäß einer besonders bevorzugten Ausführungsform ist die Gruppe Ar¹ ausgewählt aus Phenyl und Naphthyl, welche jeweils mit einem oder mehreren Resten R² substituiert sein können.

Weiterhin ist es bevorzugt, dass die beiden Gruppen Ar¹, welche in einer Gruppe der Formel (II) an ein gemeinsames N-Atom binden, gleich gewählt sind.

Erfindungsgemäß kann die Gruppe A eine Gruppe der Formel (II) darstellen. In diesem Fall ist es bevorzugt, dass die Gruppe A eine Gruppe der folgenden Formeln (II-1) bis (II-11) darstellt: 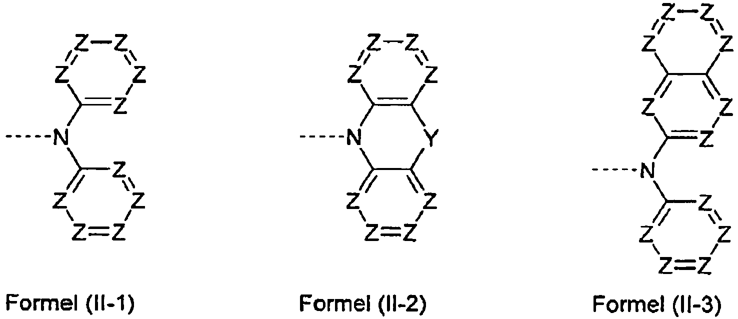 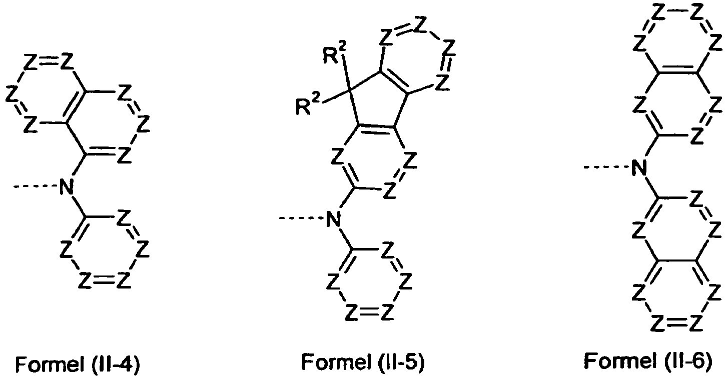 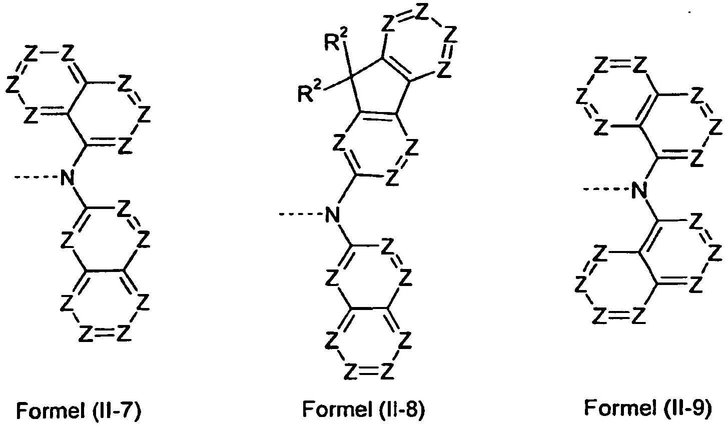 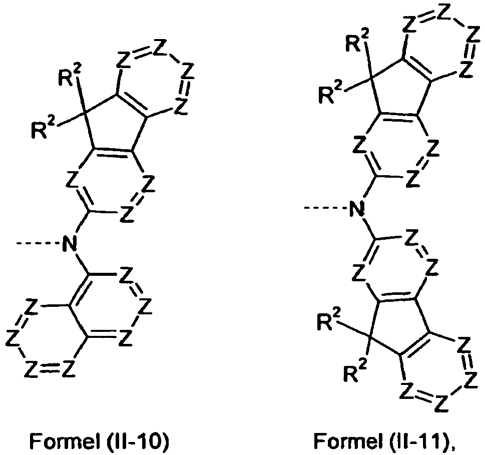 wobei R² wie oben definiert ist,
- Z: bei jedem Auftreten gleich oder verschieden CR² oder N darstellt; und
- Y: ausgewählt ist aus einer Einfachbindung, BR², C(R²)₂, Si(R²)_{2,} NR², PR², P(=O)R², O, S, S=O und S(=O)₂.

Bevorzugt sind in den oben gezeigten Formeln maximal 2 benachbarte Gruppen Z gleich N. Weiterhin ist es bevorzugt, dass 0, 1, 2 oder 3 Gruppen Z pro aromatischem Ring gleich N sind, besonders bevorzugt 0, 1 oder 2 und ganz besonders bevorzugt 0 oder 1.

Gemäß einer bevorzugten Ausführungsform ist Y ausgewählt aus einer Einfachbindung, C(R²)_{2,} NR², **O** und S.

Gemäß einer bevorzugten Ausführungsform bilden die Reste R² einer Gruppe C(R²)₂, welche für Y steht, miteinander einen Ring, so dass ein Spiro-System gebildet wird. Besonders bevorzugt wird dabei eine Gruppe der Formeln (Y-1) bis (Y-8) gebildet 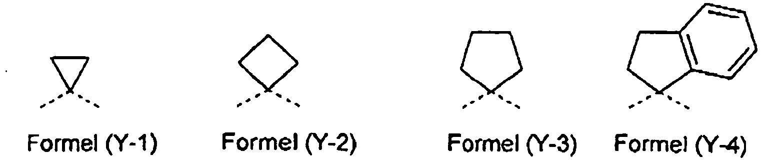 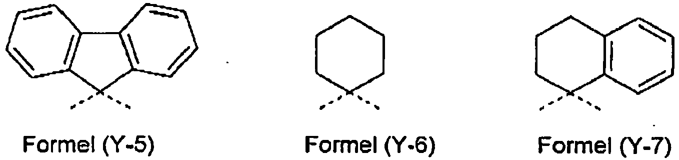 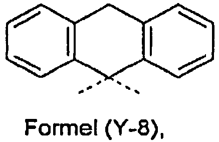 wobei die abgebildeten Gruppen in den unsubstituiert dargestellen Positionen wahlweise mit einem oder mehreren Resten R² substituiert sein können.

Erfindungsgemäß kann die Gruppe A alternativ eine Gruppe der Formel (III) darstellen. In diesem Fall stellt die Gruppe A eine Gruppe der folgenden Formeln (III-1) bis (III-3) dar: 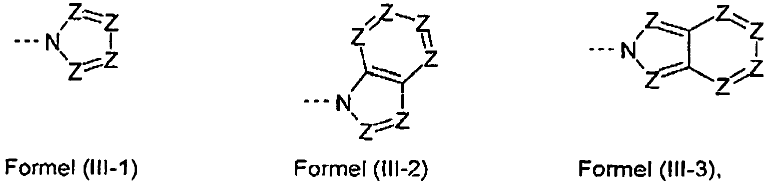 wobei
- Z: bei jedem Auftreten gleich oder verschieden CR² oder N darstellt.

Die oben genannten bevorzugten Ausführungsformen betreffend die Gruppe Z gelten auch in diesem Zusammenhang.

Besonders bevorzugt ist es, wenn A ausgewählt ist aus Pyrrol, Indol, Imidazol, Benzimidazol, Pyrazol, Benzpyrazol, 1,2,3-Triazol, Benzotriazol, 1,2,4-Triazol und Tetrazol, welches mit einem oder mehreren Resten R² substituiert sein kann.

Es soll angemerkt werden, dass die Gruppen A in der erfindungsgemäßen Verbindung der Formel (I) nicht notwendig gleich gewählt sein müssen, sondern auch verschieden sein können. Entsprechend einer bevorzugten Ausführungsform sind die Gruppen A in einer erfindungsgemäßen Verbindung der Formel (I) jedoch gleich gewählt.

Für die Gruppe R¹ ist es erfindungsgemäß bevorzugt, dass sie bei jedem Auftreten gleich oder verschieden ausgewählt ist aus F, CN, Si(R³)₃, oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, oder einer Alkenyl- oder Alkinylgruppe mit 2 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, -R³C=CR³-, Si(R³)_{2,} C=O, C=NR³, -NR³-, -O-, -S-, -C(=O)O- oder -C(=O)NR³- ersetzt sein können, oder einem aromatischen Ringsystem mit 5 bis 20 aromatischen Ringatomen, das jeweils mit einem oder mehreren Resten R³ substituiert sein kann, oder einem heteroaromatischen Ringsystem, das nicht über ein Ring-Stickstoffatom gebunden ist, und das jeweils mit einem oder mehreren Resten R³ substituiert ist.

Besonders bevorzugt ist R¹ ausgewählt aus F, CN, Si(R³)₃, geradkettigem C₁, C₂, C₃, C₄, C₅, C₆, C₇ oder C₈-Alkyl, welches mit einem oder mehreren Resten R³ substituiert sein kann, verzweigtem C₃, C₄, C₅, C₆, C₇ oder C₈-Alkyl, welches mit einem oder mehreren Resten R³ substituiert sein kann, cyclischem C₄, C₅ oder C₆-Alkyl, welches mit einem oder mehreren Resten R³ substituiert sein kann, oder C₂, C₃, C₄, C₅, C₆, C₇ oder C₈-Alkinyl, welches eine oder mehrere C-C-Dreifachbindungen enthalten kann und welches mit einem oder mehreren Resten R³ substituiert sein kann, oder einer Arylgruppe mit 6 bis 10 aromatischen Ringatomen, ganz besonders bevorzugt Phenyl, welche mit einem oder mehreren Resten R³ substituiert sein kann, oder einer Heteroarylgruppe mit 5 bis 10 aromatischen Ringatomen, welche nicht über ein Ring-Stickstoffatom gebunden ist, ganz besonders bevorzugt Pyridyl, Pyrazinyl, Pyridazinyl, Pyrimidyl und Triazinyl, welche mit einem oder mehreren Resten R³ substituiert sein kann.

Gemäß einer weiteren bevorzugten Ausführungsform sind die Reste R¹ in der erfindungsgemäßen Verbindung der Formel (I) gleich gewählt. Sie können jedoch auch unterschiedlich gewählt sein.

Bevorzugt ist der Rest R² bei jedem Auftreten gleich oder verschieden ausgewählt aus H, D, F, CN, Si(R³)₃, N(R³)₂ oder einer geradkettigen Alkyl-oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-, -R³C=CR³-, Si(R³)₂, C=O, C=NR³, -NR³-, -O-, -S-, -C(=O)O- oder -C(=O)NR³- ersetzt sein können, oder einem aromatischen oder heteroaromatischen Ringsystem mit 5 bis 20 aromatischen Ringatomen, das jeweils mit einem oder mehreren Resten R³ substituiert sein kann, wobei zwei oder mehr Reste R² miteinander verknüpft sein können und einen Ring bilden können.

Bevorzugt ist der Rest R³ bei jedem Auftreten gleich oder verschieden ausgewählt aus H, D, F, CN, Si(R⁴)₃, N(R⁴)₂ oder einer geradkettigen Alkyl-oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, C=NR⁴, -NR⁴-, -O-, -S-, -C(=O)O- oder -C(=O)NR⁴- ersetzt sein können, oder einem aromatischen oder heteroaromatischen Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

Besonders bevorzugte Gruppen A in den erfindungsgemäßen Verbindungen der Formel (I) stellen die folgenden Gruppen A-1 bis A-48 dar: 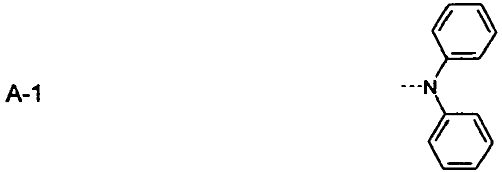 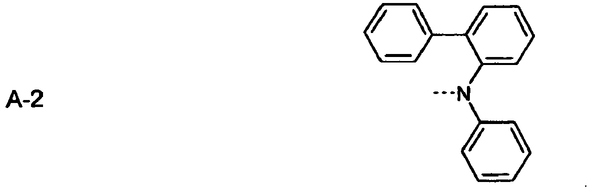 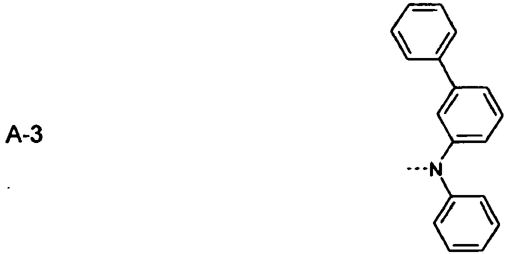 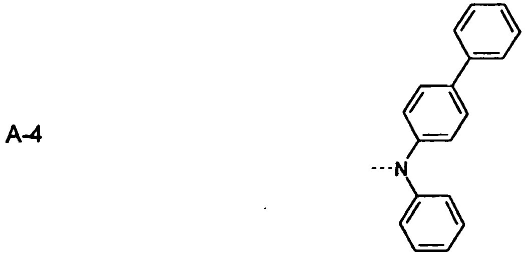 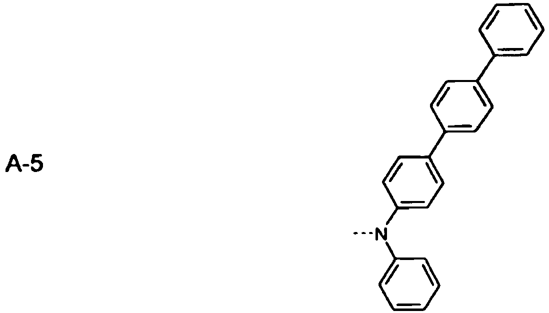 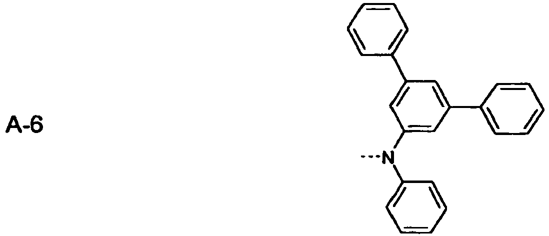 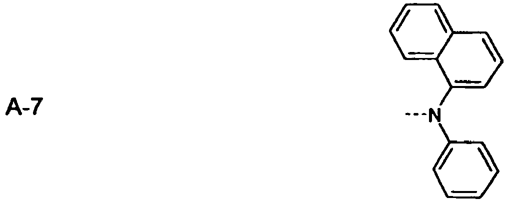 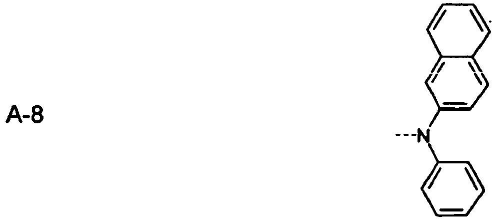 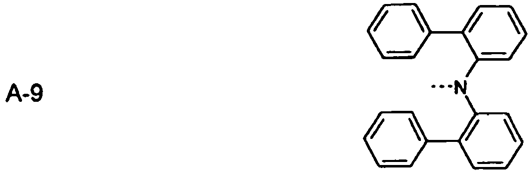 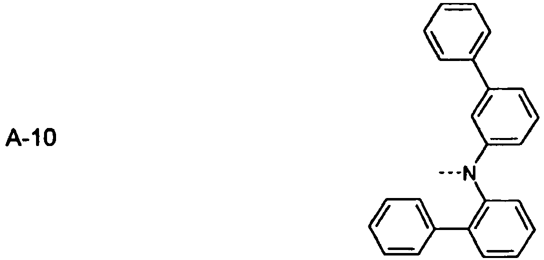 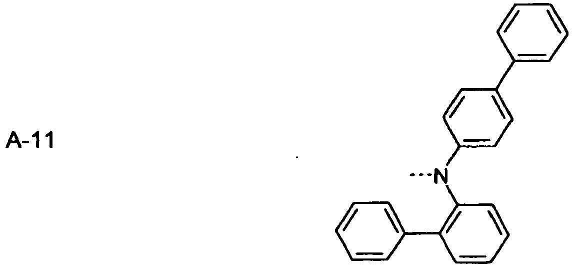 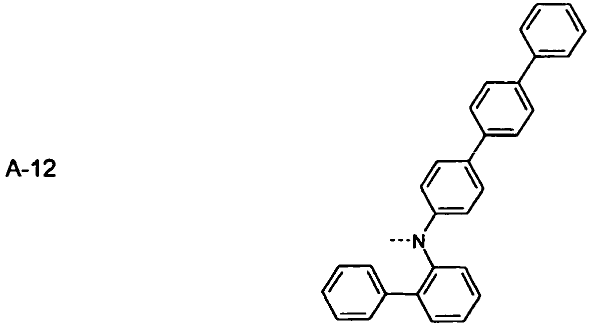 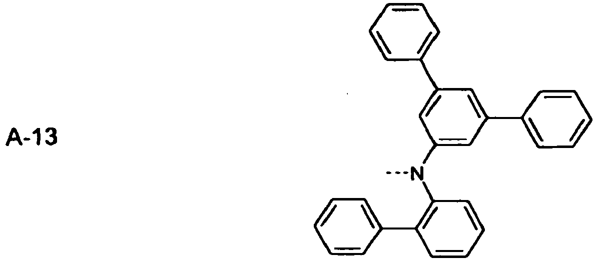 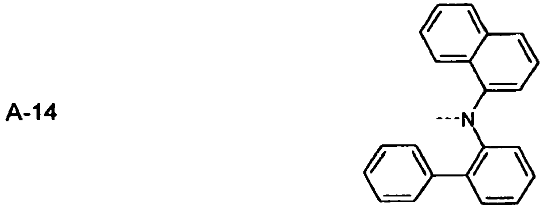 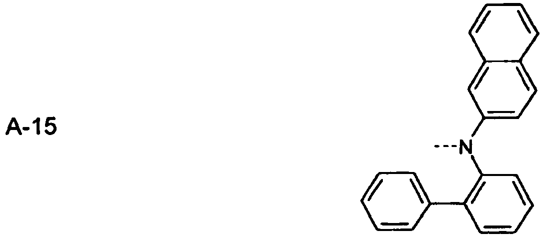 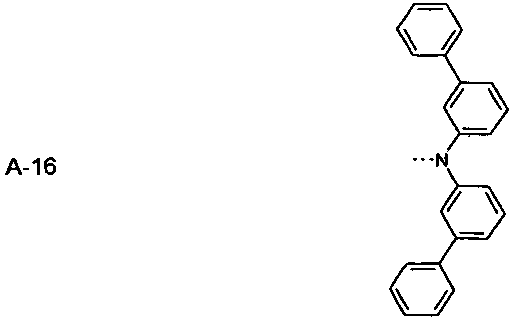 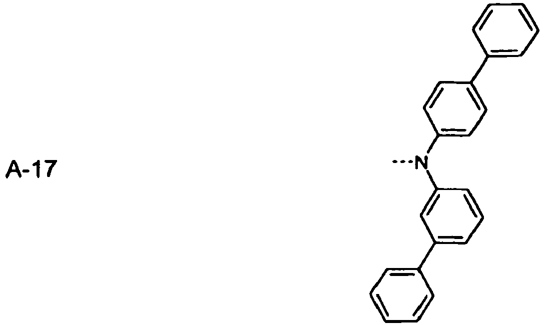 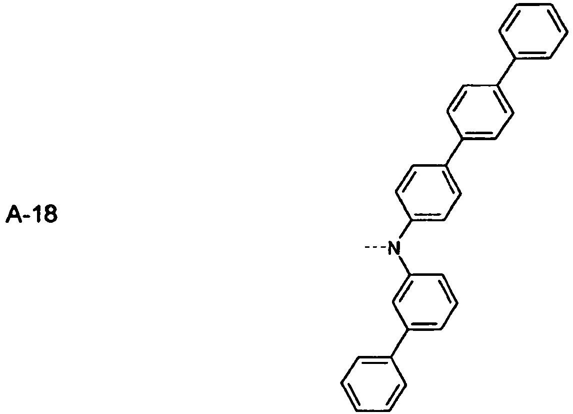 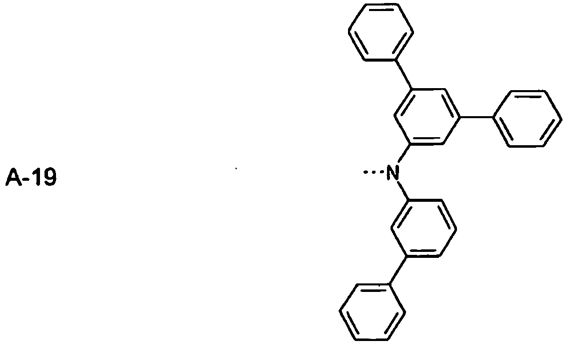 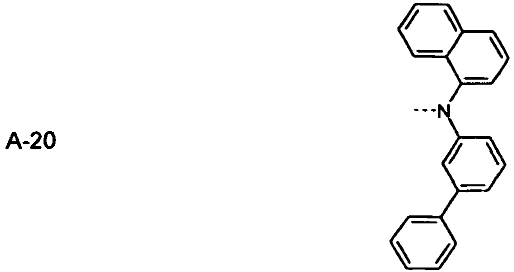 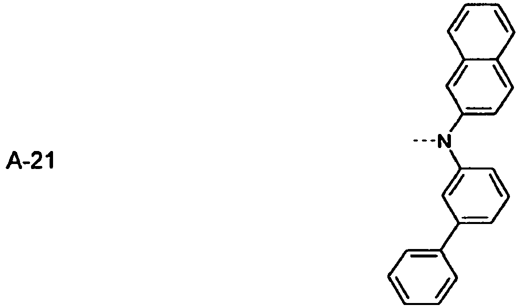 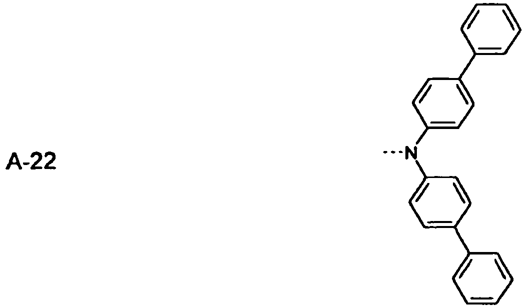 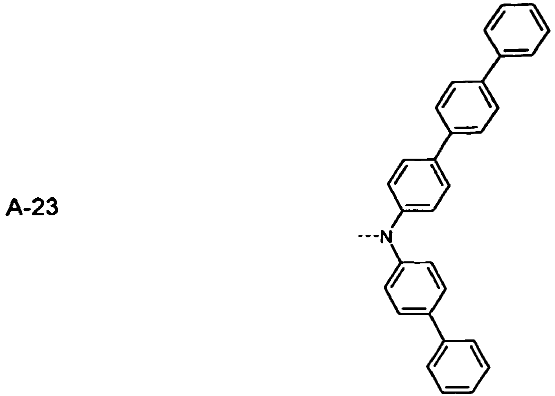 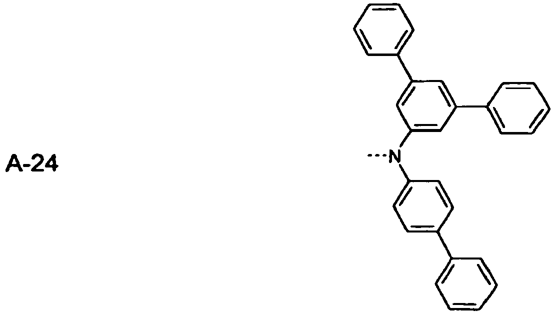 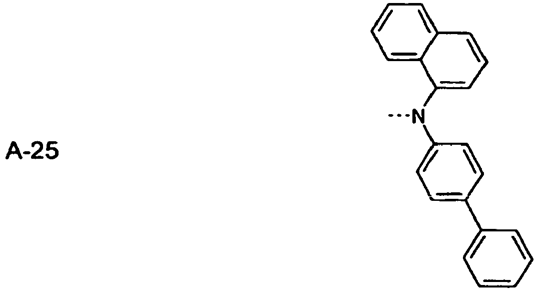 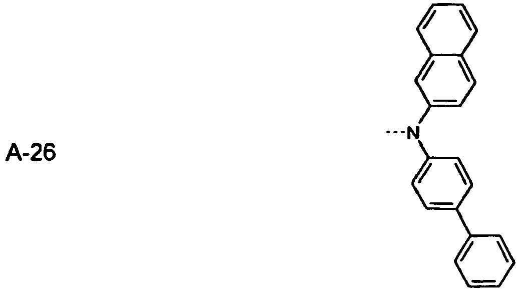 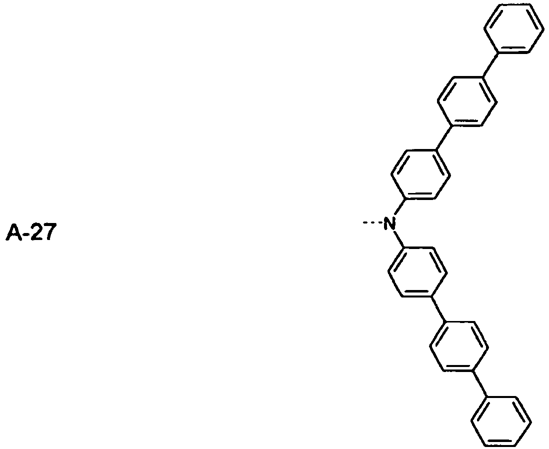 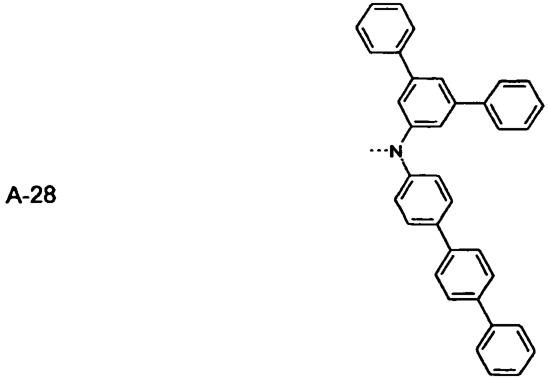 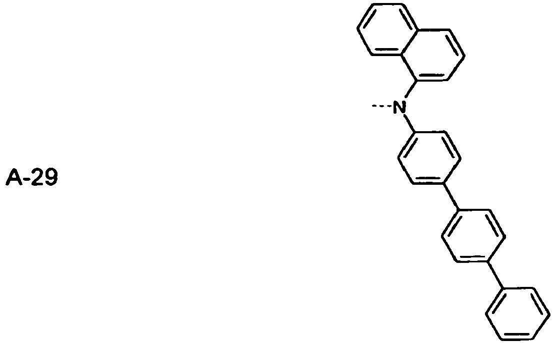 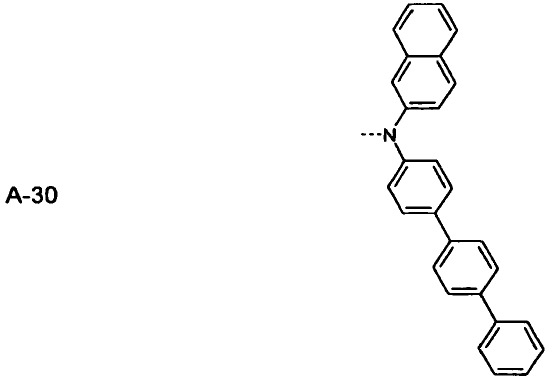 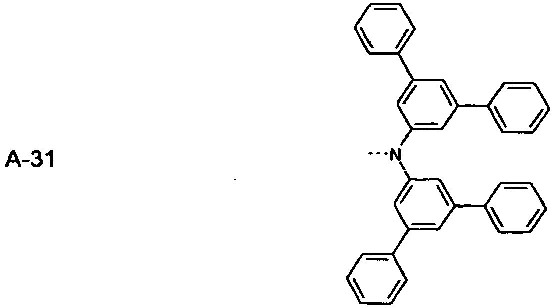 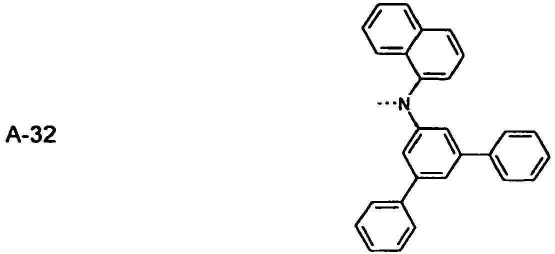 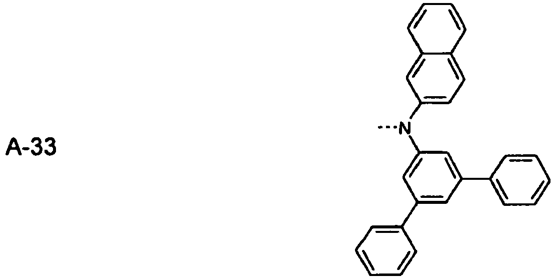 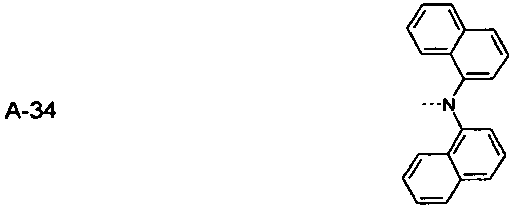 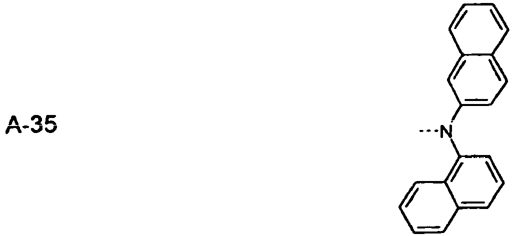 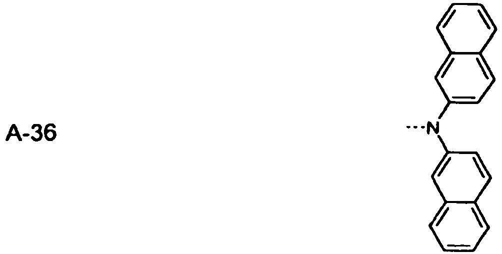 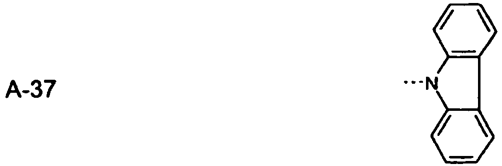 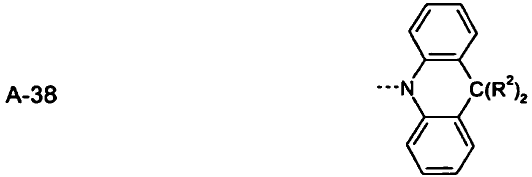 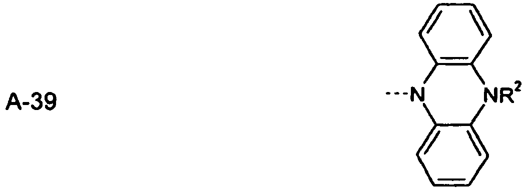 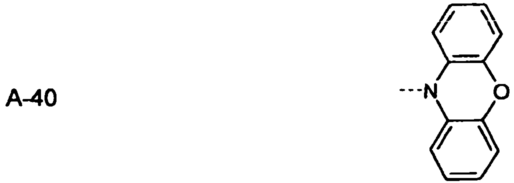 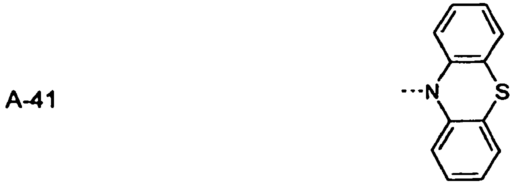 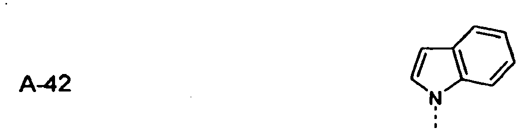 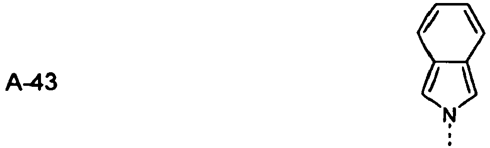 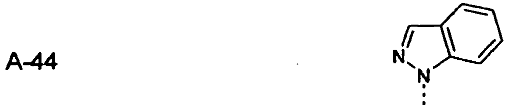 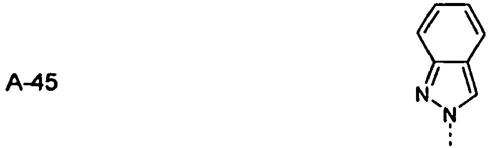 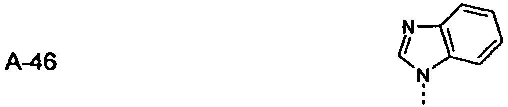 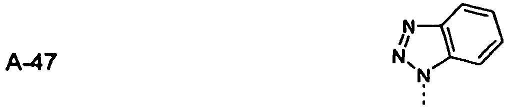 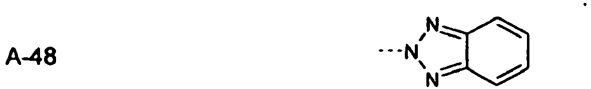 welche jeweils mit einem oder mehreren Resten R² substituiert sein können.

Besonders bevorzugte Gruppen R¹ in den erfindungsgemäßen Verbindungen der Formel (I) stellen die folgenden Gruppen R-1 bis R-8 dar:

R-1 -CH₃

R-2 -F

R-3 -CN

R-4 -CH(CH₃)₂

R-5 -C(CH₃)₃

R-6 -Ph

R-7 -CD₃

R-8 Si(CH₃)₃

Bevorzugt sind Verbindungen der untenstehenden Formel (I-3) 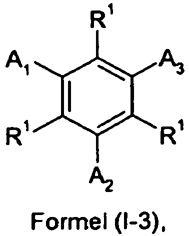 wobei A₁, A₂ und A₃ ausgewählt sind aus den oben angegebenen Resten A-1 bis A-48, und R¹ bei jedem Auftreten gleich oder verschieden ausgewählt ist aus den oben angegebenen Resten R-1 bis R-8. Bevorzugt sind dabei alle Gruppen R¹ gleich gewählt. Bevorzugt sind dabei weiterhin die Gruppen A₁, A₂ und A₃ gleich gewählt. Gemäß einer alternativen bevorzugten Ausführungsform sind genau zwei Gruppen ausgewählt aus den Gruppen A₁, A₂ und A₃ gleich gewählt, und die verbleibende Gruppe ausgewählt aus den Gruppen A₁, A₂ und A₃ ist anders gewählt.

Besonders bevorzugt sind Verbindungen der Formel (I-3), wobei die Reste R¹, A₁, A₂ und A₃ gewählt sind wie in der folgenden Tabelle angegeben.

| | R¹ | A₁ | A₂ | A₃ |
|---|---|---|---|---|
| 1 | R-1 | A-1 | A-1 | A-1 |
| 2 | R-1 | A-1 | A-1 | A-9 |
| 3 | R-1 | A-1 | A-1 | A-16 |
| 4 | R-1 | A-1 | A-1 | A-22 |
| 5 | R-1 | A-1 | A-9 | A-1 |
| 6 | R-1 | A-1 | A-9 | A-9 |
| 7 | R-1 | A-1 | A-9 | A-16 |
| 8 | R-1 | A-1 | A-9 | A-22 |
| 9 | R-1 | A-1 | A-16 | A-1 |
| 10 | R-1 | A-1 | A-16 | A-9 |
| 11 | R-1 | A-1 | A-16 | A-16 |
| 12 | R-1 | A-1 | A-16 | A-22 |
| 13 | R-1 | A-1 | A-22 | A-1 |
| 14 | R-1 | A-1 | A-22 | A-9 |
| 15 | R-1 | A-1 | A-22 | A-16 |
| 16 | R-1 | A-1 | A-22 | A-22 |
| 17 | R-1 | A-9 | A-1 | A-1 |
| 18 | R-1 | A-9 | A-1 | A-9 |
| 19 | R-1 | A-9 | A-1 | A-16 |
| 20 | R-1 | A-9 | A-1 | A-22 |
| 21 | R-1 | A-9 | A-9 | A-1 |
| 22 | R-1 | A-9 | A-9 | A-9 |
| 23 | R-1 | A-9 | A-9 | A-16 |
| 24 | R-1 | A-9 | A-9 | A-22 |
| 25 | R-1 | A-9 | A-16 | A-1 |
| 26 | R-1 | A-9 | A-16 | A-9 |
| 27 | R-1 | A-9 | A-16 | A-16 |
| 28 | R-1 | A-9 | A-16 | A-22 |
| 29 | R-1 | A-9 | A-22 | A-1 |
| 30 | R-1 | A-9 | A-22 | A-9 |
| 31 | R-1 | A-9 | A-22 | A-16 |
| 32 | R-1 | A-9 | A-22 | A-22 |
| 33 | R-1 | A-16 | A-1 | A-1 |
| 34 | R-1 | A-16 | A-1 | A-9 |
| 35 | R-1 | A-16 | A-1 | A-16 |
| 36 | R-1 | A-16 | A-1 | A-22 |
| 37 | R-1 | A-16 | A-9 | A-1 |
| 38 | R-1 | A-16 | A-9 | A-9 |
| 39 | R-1 | A-16 | A-9 | A-16 |
| 40 | R-1 | A-16 | A-9 | A-22 |
| 41 | R-1 | A-16 | A-16 | A-1 |
| 42 | R-1 | A-16 | A-16 | A-9 |
| 43 | R-1 | A-16 | A-16 | A-16 |
| 44 | R-1 | A-16 | A-16 | A-22 |
| 45 | R-1 | A-16 | A-22 | A-1 |
| 46 | R-1 | A-16 | A-22 | A-9 |
| 47 | R-1 | A-16 | A-22 | A-16 |
| 48 | R-1 | A-16 | A-22 | A-22 |
| 49 | R-1 | A-22 | A-1 | A-1 |
| 50 | R-1 | A-22 | A-1 | A-9 |
| 51 | R-1 | A-22 | A-1 | A-16 |
| 52 | R-1 | A-22 | A-1 | A-22 |
| 53 | R-1 | A-22 | A-9 | A-1 |
| 54 | R-1 | A-22 | A-9 | A-9 |
| 55 | R-1 | A-22 | A-9 | A-16 |
| 56 | R-1 | A-22 | A-9 | A-22 |
| 57 | R-1 | A-22 | A-16 | A-1 |
| 58 | R-1 | A-22 | A-16 | A-9 |
| 59 | R-1 | A-22 | A-16 | A-16 |
| 60 | R-1 | A-22 | A-16 | A-22 |
| 61 | R-1 | A-22 | A-22 | A-1 |
| 62 | R-1 | A-22 | A-22 | A-9 |
| 63 | R-1 | A-22 | A-22 | A-16 |
| 64 | R-1 | A-22 | A-22 | A-22 |
| 65 | R-2 | A-1 | A-1 | A-1 |
| 66 | R-2 | A-1 | A-1 | A-9 |
| 67 | R-2 | A-1 | A-1 | A-16 |
| 68 | R-2 | A-1 | A-1 | A-22 |
| 69 | R-2 | A-1 | A-9 | A-1 |
| 70 | R-2 | A-1 | A-9 | A-9 |
| 71 | R-2 | A-1 | A-9 | A-16 |
| 72 | R-2 | A-1 | A-9 | A-22 |
| 73 | R-2 | A-1 | A-16 | A-1 |
| 74 | R-2 | A-1 | A-16 | A-9 |
| 75 | R-2 | A-1 | A-16 | A-16 |
| 76 | R-2 | A-1 | A-16 | A-22 |
| 77 | R-2 | A-1 | A-22 | A-1 |
| 78 | R-2 | A-1 | A-22 | A-9 |
| 79 | R-2 | A-1 | A-22 | A-16 |
| 80 | R-2 | A-1 | A-22 | A-22 |
| 81 | R-2 | A-9 | A-1 | A-1 |
| 82 | R-2 | A-9 | A-1 | A-9 |
| 83 | R-2 | A-9 | A-1 | A-16 |
| 84 | R-2 | A-9 | A-1 | A-22 |
| 85 | R-2 | A-9 | A-9 | A-1 |
| 86 | R-2 | A-9 | A-9 | A-9 |
| 87 | R-2 | A-9 | A-9 | A-16 |
| 88 | R-2 | A-9 | A-9 | A-22 |
| 89 | R-2 | A-9 | A-16 | A-1 |
| 90 | R-2 | A-9 | A-16 | A-9 |
| 91 | R-2 | A-9 | A-16 | A-16 |
| 92 | R-2 | A-9 | A-16 | A-22 |
| 93 | R-2 | A-9 | A-22 | A-1 |
| 94 | R-2 | A-9 | A-22 | A-9 |
| 95 | R-2 | A-9 | A-22 | A-16 |
| 96 | R-2 | A-9 | A-22 | A-22 |
| 97 | R-2 | A-16 | A-1 | A-1 |
| 98 | R-2 | A-16 | A-1 | A-9 |
| 99 | R-2 | A-16 | A-1 | A-16 |
| 100 | R-2 | A-16 | A-1 | A-22 |
| 101 | R-2 | A-16 | A-9 | A-1 |
| 102 | R-2 | A-16 | A-9 | A-9 |
| 103 | R-2 | A-16 | A-9 | A-16 |
| 104 | R-2 | A-16 | A-9 | A-22 |
| 105 | R-2 | A-16 | A-16 | A-1 |
| 106 | R-2 | A-16 | A-16 | A-9 |
| 107 | R-2 | A-16 | A-16 | A-16 |
| 108 | R-2 | A-16 | A-16 | A-22 |
| 109 | R-2 | A-16 | A-22 | A-1 |
| 110 | R-2 | A-16 | A-22 | A-9 |
| 111 | R-2 | A-16 | A-22 | A-16 |
| 112 | R-2 | A-16 | A-22 | A-22 |
| 113 | R-2 | A-22 | A-1 | A-1 |
| 114 | R-2 | A-22 | A-1 | A-9 |
| 115 | R-2 | A-22 | A-1 | A-16 |
| 116 | R-2 | A-22 | A-1 | A-22 |
| 117 | R-2 | A-22 | A-9 | A-1 |
| 118 | R-2 | A-22 | A-9 | A-9 |
| 119 | R-2 | A-22 | A-9 | A-16 |
| 120 | R-2 | A-22 | A-9 | A-22 |
| 121 | R-2 | A-22 | A-16 | A-1 |
| 122 | R-2 | A-22 | A-16 | A-9 |
| 123 | R-2 | A-22 | A-16 | A-16 |
| 124 | R-2 | A-22 | A-16 | A-22 |
| 125 | R-2 | A-22 | A-22 | A-1 |
| 126 | R-2 | A-22 | A-22 | A-9 |
| 127 | R-2 | A-22 | A-22 | A-16 |
| 128 | R-2 | A-22 | A-22 | A-22 |
| 129 | R-3 | A-1 | A-1 | A-1 |
| 130 | R-3 | A-1 | A-1 | A-9 |
| 131 | R-3 | A-1 | A-1 | A-16 |
| 132 | R-3 | A-1 | A-1 | A-22 |
| 133 | R-3 | A-1 | A-9 | A-1 |
| 134 | R-3 | A-1 | A-9 | A-9 |
| 135 | R-3 | A-1 | A-9 | A-16 |
| 136 | R-3 | A-1 | A-9 | A-22 |
| 137 | R-3 | A-1 | A-16 | A-1 |
| 138 | R-3 | A-1 | A-16 | A-9 |
| 139 | R-3 | A-1 | A-16 | A-16 |
| 140 | R-3 | A-1 | A-16 | A-22 |
| 141 | R-3 | A-1 | A-22 | A-1 |
| 142 | R-3 | A-1 | A-22 | A-9 |
| 143 | R-3 | A-1 | A-22 | A-16 |
| 144 | R-3 | A-1 | A-22 | A-22 |
| 145 | R-3 | A-9 | A-1 | A-1 |
| 146 | R-3 | A-9 | A-1 | A-9 |
| 147 | R-3 | A-9 | A-1 | A-16 |
| 148 | R-3 | A-9 | A-1 | A-22 |
| 149 | R-3 | A-9 | A-9 | A-1 |
| 150 | R-3 | A-9 | A-9 | A-9 |
| 151 | R-3 | A-9 | A-9 | A-16 |
| 152 | R-3 | A-9 | A-9 | A-22 |
| 153 | R-3 | A-9 | A-16 | A-1 |
| 154 | R-3 | A-9 | A-16 | A-9 |
| 155 | R-3 | A-9 | A-16 | A-16 |
| 156 | R-3 | A-9 | A-16 | A-22 |
| 157 | R-3 | A-9 | A-22 | A-1 |
| 158 | R-3 | A-9 | A-22 | A-9 |
| 159 | R-3 | A-9 | A-22 | A-16 |
| 160 | R-3 | A-9 | A-22 | A-22 |
| 161 | R-3 | A-16 | A-1 | A-1 |
| 162 | R-3 | A-16 | A-1 | A-9 |
| 163 | R-3 | A-16 | A-1 | A-16 |
| 164 | R-3 | A-16 | A-1 | A-22 |
| 165 | R-3 | A-16 | A-9 | A-1 |
| 166 | R-3 | A-16 | A-9 | A-9 |
| 167 | R-3 | A-16 | A-9 | A-16 |
| 168 | R-3 | A-16 | A-9 | A-22 |
| 169 | R-3 | A-16 | A-16 | A-1 |
| 170 | R-3 | A-16 | A-16 | A-9 |
| 171 | R-3 | A-16 | A-16 | A-16 |
| 172 | R-3 | A-16 | A-16 | A-22 |
| 173 | R-3 | A-16 | A-22 | A-1 |
| 174 | R-3 | A-16 | A-22 | A-9 |
| 175 | R-3 | A-16 | A-22 | A-16 |
| 176 | R-3 | A-16 | A-22 | A-22 |
| 177 | R-3 | A-22 | A-1 | A-1 |
| 178 | R-3 | A-22 | A-1 | A-9 |
| 179 | R-3 | A-22 | A-1 | A-16 |
| 180 | R-3 | A-22 | A-1 | A-22 |
| 181 | R-3 | A-22 | A-9 | A-1 |
| 182 | R-3 | A-22 | A-9 | A-9 |
| 183 | R-3 | A-22 | A-9 | A-16 |
| 184 | R-3 | A-22 | A-9 | A-22 |
| 185 | R-3 | A-22 | A-16 | A-1 |
| 186 | R-3 | A-22 | A-16 | A-9 |
| 187 | R-3 | A-22 | A-16 | A-16 |
| 188 | R-3 | A-22 | A-16 | A-22 |
| 189 | R-3 | A-22 | A-22 | A-1 |
| 190 | R-3 | A-22 | A-22 | A-9 |
| 191 | R-3 | A-22 | A-22 | A-16 |
| 192 | R-3 | A-22 | A-22 | A-22 |
| 193 | R-4 | A-1 | A-1 | A-1 |
| 194 | R-4 | A-1 | A-1 | A-9 |
| 195 | R-4 | A-1 | A-1 | A-16 |
| 196 | R-4 | A-1 | A-1 | A-22 |
| 197 | R-4 | A-1 | A-9 | A-1 |
| 198 | R-4 | A-1 | A-9 | A-9 |
| 199 | R-4 | A-1 | A-9 | A-16 |
| 200 | R-4 | A-1 | A-9 | A-22 |
| 201 | R-4 | A-1 | A-16 | A-1 |
| 202 | R-4 | A-1 | A-16 | A-9 |
| 203 | R-4 | A-1 | A-16 | A-16 |
| 204 | R-4 | A-1 | A-16 | A-22 |
| 205 | R-4 | A-1 | A-22 | A-1 |
| 206 | R-4 | A-1 | A-22 | A-9 |
| 207 | R-4 | A-1 | A-22 | A-16 |
| 208 | R-4 | A-1 | A-22 | A-22 |
| 209 | R-4 | A-9 | A-1 | A-1 |
| 210 | R-4 | A-9 | A-1 | A-9 |
| 211 | R-4 | A-9 | A-1 | A-16 |
| 212 | R-4 | A-9 | A-1 | A-22 |
| 213 | R-4 | A-9 | A-9 | A-1 |
| 214 | R-4 | A-9 | A-9 | A-9 |
| 215 | R-4 | A-9 | A-9 | A-16 |
| 216 | R-4 | A-9 | A-9 | A-22 |
| 217 | R-4 | A-9 | A-16 | A-1 |
| 218 | R-4 | A-9 | A-16 | A-9 |
| 219 | R-4 | A-9 | A-16 | A-16 |
| 220 | R-4 | A-9 | A-16 | A-22 |
| 221 | R-4 | A-9 | A-22 | A-1 |
| 222 | R-4 | A-9 | A-22 | A-9 |
| 223 | R-4 | A-9 | A-22 | A-16 |
| 224 | R-4 | A-9 | A-22 | A-22 |
| 225 | R-4 | A-16 | A-1 | A-1 |
| 226 | R-4 | A-16 | A-1 | A-9 |
| 227 | R-4 | A-16 | A-1 | A-16 |
| 228 | R-4 | A-16 | A-1 | A-22 |
| 229 | R-4 | A-16 | A-9 | A-1 |
| 230 | R-4 | A-16 | A-9 | A-9 |
| 231 | R-4 | A-16 | A-9 | A-16 |
| 232 | R-4 | A-16 | A-9 | A-22 |
| 233 | R-4 | A-16 | A-16 | A-1 |
| 234 | R-4 | A-16 | A-16 | A-9 |
| 235 | R-4 | A-16 | A-16 | A-16 |
| 236 | R-4 | A-16 | A-16 | A-22 |
| 237 | R-4 | A-16 | A-22 | A-1 |
| 238 | R-4 | A-16 | A-22 | A-9 |
| 239 | R-4 | A-16 | A-22 | A-16 |
| 240 | R-4 | A-16 | A-22 | A-22 |
| 241 | R-4 | A-22 | A-1 | A-1 |
| 242 | R-4 | A-22 | A-1 | A-9 |
| 243 | R-4 | A-22 | A-1 | A-16 |
| 244 | R-4 | A-22 | A-1 | A-22 |
| 245 | R-4 | A-22 | A-9 | A-1 |
| 246 | R-4 | A-22 | A-9 | A-9 |
| 247 | R-4 | A-22 | A-9 | A-16 |
| 248 | R-4 | A-22 | A-9 | A-22 |
| 249 | R-4 | A-22 | A-16 | A-1 |
| 250 | R-4 | A-22 | A-16 | A-9 |
| 251 | R-4 | A-22 | A-16 | A-16 |
| 252 | R-4 | A-22 | A-16 | A-22 |
| 253 | R-4 | A-22 | A-22 | A-1 |
| 254 | R-4 | A-22 | A-22 | A-9 |
| 255 | R-4 | A-22 | A-22 | A-16 |
| 256 | R-4 | A-22 | A-22 | A-22 |
| 257 | R-5 | A-1 | A-1 | A-1 |
| 258 | R-5 | A-1 | A-1 | A-9 |
| 259 | R-5 | A-1 | A-1 | A-16 |
| 260 | R-5 | A-1 | A-1 | A-22 |
| 261 | R-5 | A-1 | A-9 | A-1 |
| 262 | R-5 | A-1 | A-9 | A-9 |
| 263 | R-5 | A-1 | A-9 | A-16 |
| 264 | R-5 | A-1 | A-9 | A-22 |
| 265 | R-5 | A-1 | A-16 | A-1 |
| 266 | R-5 | A-1 | A-16 | A-9 |
| 267 | R-5 | A-1 | A-16 | A-16 |
| 268 | R-5 | A-1 | A-16 | A-22 |
| 269 | R-5 | A-1 | A-22 | A-1 |
| 270 | R-5 | A-1 | A-22 | A-9 |
| 271 | R-5 | A-1 | A-22 | A-16 |
| 272 | R-5 | A-1 | A-22 | A-22 |
| 273 | R-5 | A-9 | A-1 | A-1 |
| 274 | R-5 | A-9 | A-1 | A-9 |
| 275 | R-5 | A-9 | A-1 | A-16 |
| 276 | R-5 | A-9 | A-1 | A-22 |
| 277 | R-5 | A-9 | A-9 | A-1 |
| 278 | R-5 | A-9 | A-9 | A-9 |
| 279 | R-5 | A-9 | A-9 | A-16 |
| 280 | R-5 | A-9 | A-9 | A-22 |
| 281 | R-5 | A-9 | A-16 | A-1 |
| 282 | R-5 | A-9 | A-16 | A-9 |
| 283 | R-5 | A-9 | A-16 | A-16 |
| 284 | R-5 | A-9 | A-16 | A-22 |
| 285 | R-5 | A-9 | A-22 | A-1 |
| 286 | R-5 | A-9 | A-22 | A-9 |
| 287 | R-5 | A-9 | A-22 | A-16 |
| 288 | R-5 | A-9 | A-22 | A-22 |
| 289 | R-5 | A-16 | A-1 | A-1 |
| 290 | R-5 | A-16 | A-1 | A-9 |
| 291 | R-5 | A-16 | A-1 | A-16 |
| 292 | R-5 | A-16 | A-1 | A-22 |
| 293 | R-5 | A-16 | A-9 | A-1 |
| 294 | R-5 | A-16 | A-9 | A-9 |
| 295 | R-5 | A-16 | A-9 | A-16 |
| 296 | R-5 | A-16 | A-9 | A-22 |
| 297 | R-5 | A-16 | A-16 | A-1 |
| 298 | R-5 | A-16 | A-16 | A-9 |
| 299 | R-5 | A-16 | A-16 | A-16 |
| 300 | R-5 | A-16 | A-16 | A-22 |
| 301 | R-5 | A-16 | A-22 | A-1 |
| 302 | R-5 | A-16 | A-22 | A-9 |
| 303 | R-5 | A-16 | A-22 | A-16 |
| 304 | R-5 | A-16 | A-22 | A-22 |
| 305 | R-5 | A-22 | A-1 | A-1 |
| 306 | R-5 | A-22 | A-1 | A-9 |
| 307 | R-5 | A-22 | A-1 | A-16 |
| 308 | R-5 | A-22 | A-1 | A-22 |
| 309 | R-5 | A-22 | A-9 | A-1 |
| 310 | R-5 | A-22 | A-9 | A-9 |
| 311 | R-5 | A-22 | A-9 | A-16 |
| 312 | R-5 | A-22 | A-9 | A-22 |
| 313 | R-5 | A-22 | A-16 | A-1 |
| 314 | R-5 | A-22 | A-16 | A-9 |
| 315 | R-5 | A-22 | A-16 | A-16 |
| 316 | R-5 | A-22 | A-16 | A-22 |
| 317 | R-5 | A-22 | A-22 | A-1 |
| 318 | R-5 | A-22 | A-22 | A-9 |
| 319 | R-5 | A-22 | A-22 | A-16 |
| 320 | R-5 | A-22 | A-22 | A-22 |
| 321 | R-6 | A-1 | A-1 | A-1 |
| 322 | R-6 | A-1 | A-1 | A-9 |
| 323 | R-6 | A-1 | A-1 | A-16 |
| 324 | R-6 | A-1 | A-1 | A-22 |
| 325 | R-6 | A-1 | A-9 | A-1 |
| 326 | R-6 | A-1 | A-9 | A-9 |
| 327 | R-6 | A-1 | A-9 | A-16 |
| 328 | R-6 | A-1 | A-9 | A-22 |
| 329 | R-6 | A-1 | A-16 | A-1 |
| 330 | R-6 | A-1 | A-16 | A-9 |
| 331 | R-6 | A-1 | A-16 | A-16 |
| 332 | R-6 | A-1 | A-16 | A-22 |
| 333 | R-6 | A-1 | A-22 | A-1 |
| 334 | R-6 | A-1 | A-22 | A-9 |
| 335 | R-6 | A-1 | A-22 | A-16 |
| 336 | R-6 | A-1 | A-22 | A-22 |
| 337 | R-6 | A-9 | A-1 | A-1 |
| 338 | R-6 | A-9 | A-1 | A-9 |
| 339 | R-6 | A-9 | A-1 | A-16 |
| 340 | R-6 | A-9 | A-1 | A-22 |
| 341 | R-6 | A-9 | A-9 | A-1 |
| 342 | R-6 | A-9 | A-9 | A-9 |
| 343 | R-6 | A-9 | A-9 | A-16 |
| 344 | R-6 | A-9 | A-9 | A-22 |
| 345 | R-6 | A-9 | A-16 | A-1 |
| 346 | R-6 | A-9 | A-16 | A-9 |
| 347 | R-6 | A-9 | A-16 | A-16 |
| 348 | R-6 | A-9 | A-16 | A-22 |
| 349 | R-6 | A-9 | A-22 | A-1 |
| 350 | R-6 | A-9 | A-22 | A-9 |
| 351 | R-6 | A-9 | A-22 | A-16 |
| 352 | R-6 | A-9 | A-22 | A-22 |
| 353 | R-6 | A-16 | A-1 | A-1 |
| 354 | R-6 | A-16 | A-1 | A-9 |
| 355 | R-6 | A-16 | A-1 | A-16 |
| 356 | R-6 | A-16 | A-1 | A-22 |
| 357 | R-6 | A-16 | A-9 | A-1 |
| 358 | R-6 | A-16 | A-9 | A-9 |
| 359 | R-6 | A-16 | A-9 | A-16 |
| 360 | R-6 | A-16 | A-9 | A-22 |
| 361 | R-6 | A-16 | A-16 | A-1 |
| 362 | R-6 | A-16 | A-16 | A-9 |
| 363 | R-6 | A-16 | A-16 | A-16 |
| 364 | R-6 | A-16 | A-16 | A-22 |
| 365 | R-6 | A-16 | A-22 | A-1 |
| 366 | R-6 | A-16 | A-22 | A-9 |
| 367 | R-6 | A-16 | A-22 | A-16 |
| 368 | R-6 | A-16 | A-22 | A-22 |
| 369 | R-6 | A-22 | A-1 | A-1 |
| 370 | R-6 | A-22 | A-1 | A-9 |
| 371 | R-6 | A-22 | A-1 | A-16 |
| 372 | R-6 | A-22 | A-1 | A-22 |
| 373 | R-6 | A-22 | A-9 | A-1 |
| 374 | R-6 | A-22 | A-9 | A-9 |
| 375 | R-6 | A-22 | A-9 | A-16 |
| 376 | R-6 | A-22 | A-9 | A-22 |
| 377 | R-6 | A-22 | A-16 | A-1 |
| 378 | R-6 | A-22 | A-16 | A-9 |
| 379 | R-6 | A-22 | A-16 | A-16 |
| 380 | R-6 | A-22 | A-16 | A-22 |
| 381 | R-6 | A-22 | A-22 | A-1 |
| 382 | R-6 | A-22 | A-22 | A-9 |
| 383 | R-6 | A-22 | A-22 | A-16 |
| 384 | R-6 | A-22 | A-22 | A-22 |
| 385 | R-7 | A-1 | A-1 | A-1 |
| 386 | R-7 | A-1 | A-1 | A-9 |
| 387 | R-7 | A-1 | A-1 | A-16 |
| 388 | R-7 | A-1 | A-1 | A-22 |
| 389 | R-7 | A-1 | A-9 | A-1 |
| 390 | R-7 | A-1 | A-9 | A-9 |
| 391 | R-7 | A-1 | A-9 | A-16 |
| 392 | R-7 | A-1 | A-9 | A-22 |
| 393 | R-7 | A-1 | A-16 | A-1 |
| 394 | R-7 | A-1 | A-16 | A-9 |
| 395 | R-7 | A-1 | A-16 | A-16 |
| 396 | R-7 | A-1 | A-16 | A-22 |
| 397 | R-7 | A-1 | A-22 | A-1 |
| 398 | R-7 | A-1 | A-22 | A-9 |
| 399 | R-7 | A-1 | A-22 | A-16 |
| 400 | R-7 | A-1 | A-22 | A-22 |
| 401 | R-7 | A-9 | A-1 | A-1 |
| 402 | R-7 | A-9 | A-1 | A-9 |
| 403 | R-7 | A-9 | A-1 | A-16 |
| 404 | R-7 | A-9 | A-1 | A-22 |
| 405 | R-7 | A-9 | A-9 | A-1 |
| 406 | R-7 | A-9 | A-9 | A-9 |
| 407 | R-7 | A-9 | A-9 | A-16 |
| 408 | R-7 | A-9 | A-9 | A-22 |
| 409 | R-7 | A-9 | A-16 | A-1 |
| 410 | R-7 | A-9 | A-16 | A-9 |
| 411 | R-7 | A-9 | A-16 | A-16 |
| 412 | R-7 | A-9 | A-16 | A-22 |
| 413 | R-7 | A-9 | A-22 | A-1 |
| 414 | R-7 | A-9 | A-22 | A-9 |
| 415 | R-7 | A-9 | A-22 | A-16 |
| 416 | R-7 | A-9 | A-22 | A-22 |
| 417 | R-7 | A-16 | A-1 | A-1 |
| 418 | R-7 | A-16 | A-1 | A-9 |
| 419 | R-7 | A-16 | A-1 | A-16 |
| 420 | R-7 | A-16 | A-1 | A-22 |
| 421 | R-7 | A-16 | A-9 | A-1 |
| 422 | R-7 | A-16 | A-9 | A-9 |
| 423 | R-7 | A-16 | A-9 | A-16 |
| 424 | R-7 | A-16 | A-9 | A-22 |
| 425 | R-7 | A-16 | A-16 | A-1 |
| 426 | R-7 | A-16 | A-16 | A-9 |
| 427 | R-7 | A-16 | A-16 | A-16 |
| 428 | R-7 | A-16 | A-16 | A-22 |
| 429 | R-7 | A-16 | A-22 | A-1 |
| 430 | R-7 | A-16 | A-22 | A-9 |
| 431 | R-7 | A-16 | A-22 | A-16 |
| 432 | R-7 | A-16 | A-22 | A-22 |
| 433 | R-7 | A-22 | A-1 | A-1 |
| 434 | R-7 | A-22 | A-1 | A-9 |
| 435 | R-7 | A-22 | A-1 | A-16 |
| 436 | R-7 | A-22 | A-1 | A-22 |
| 437 | R-7 | A-22 | A-9 | A-1 |
| 438 | R-7 | A-22 | A-9 | A-9 |
| 439 | R-7 | A-22 | A-9 | A-16 |
| 440 | R-7 | A-22 | A-9 | A-22 |
| 441 | R-7 | A-22 | A-16 | A-1 |
| 442 | R-7 | A-22 | A-16 | A-9 |
| 443 | R-7 | A-22 | A-16 | A-16 |
| 444 | R-7 | A-22 | A-16 | A-22 |
| 445 | R-7 | A-22 | A-22 | A-1 |
| 446 | R-7 | A-22 | A-22 | A-9 |
| 447 | R-7 | A-22 | A-22 | A-16 |
| 448 | R-7 | A-22 | A-22 | A-22 |
| 449 | R-8 | A-1 | A-1 | A-1 |
| 450 | R-8 | A-1 | A-1 | A-9 |
| 451 | R-8 | A-1 | A-1 | A-16 |
| 452 | R-8 | A-1 | A-1 | A-22 |
| 453 | R-8 | A-1 | A-9 | A-1 |
| 454 | R-8 | A-1 | A-9 | A-9 |
| 455 | R-8 | A-1 | A-9 | A-16 |
| 456 | R-8 | A-1 | A-9 | A-22 |
| 457 | R-8 | A-1 | A-16 | A-1 |
| 458 | R-8 | A-1 | A-16 | A-9 |
| 459 | R-8 | A-1 | A-16 | A-16 |
| 460 | R-8 | A-1 | A-16 | A-22 |
| 461 | R-8 | A-1 | A-22 | A-1 |
| 462 | R-8 | A-1 | A-22 | A-9 |
| 463 | R-8 | A-1 | A-22 | A-16 |
| 464 | R-8 | A-1 | A-22 | A-22 |
| 465 | R-8 | A-9 | A-1 | A-1 |
| 466 | R-8 | A-9 | A-1 | A-9 |
| 467 | R-8 | A-9 | A-1 | A-16 |
| 468 | R-8 | A-9 | A-1 | A-22 |
| 469 | R-8 | A-9 | A-9 | A-1 |
| 470 | R-8 | A-9 | A-9 | A-9 |
| 471 | R-8 | A-9 | A-9 | A-16 |
| 472 | R-8 | A-9 | A-9 | A-22 |
| 473 | R-8 | A-9 | A-16 | A-1 |
| 474 | R-8 | A-9 | A-16 | A-9 |
| 475 | R-8 | A-9 | A-16 | A-16 |
| 476 | R-8 | A-9 | A-16 | A-22 |
| 477 | R-8 | A-9 | A-22 | A-1 |
| 478 | R-8 | A-9 | A-22 | A-9 |
| 479 | R-8 | A-9 | A-22 | A-16 |
| 480 | R-8 | A-9 | A-22 | A-22 |
| 481 | R-8 | A-16 | A-1 | A-1 |
| 482 | R-8 | A-16 | A-1 | A-9 |
| 483 | R-8 | A-16 | A-1 | A-16 |
| 484 | R-8 | A-16 | A-1 | A-22 |
| 485 | R-8 | A-16 | A-9 | A-1 |
| 486 | R-8 | A-16 | A-9 | A-9 |
| 487 | R-8 | A-16 | A-9 | A-16 |
| 488 | R-8 | A-16 | A-9 | A-22 |
| 489 | R-8 | A-16 | A-16 | A-1 |
| 490 | R-8 | A-16 | A-16 | A-9 |
| 491 | R-8 | A-16 | A-16 | A-16 |
| 492 | R-8 | A-16 | A-16 | A-22 |
| 493 | R-8 | A-16 | A-22 | A-1 |
| 494 | R-8 | A-16 | A-22 | A-9 |
| 495 | R-8 | A-16 | A-22 | A-16 |
| 496 | R-8 | A-16 | A-22 | A-22 |
| 497 | R-8 | A-22 | A-1 | A-1 |
| 498 | R-8 | A-22 | A-1 | A-9 |
| 499 | R-8 | A-22 | A-1 | A-16 |
| 500 | R-8 | A-22 | A-1 | A-22 |
| 501 | R-8 | A-22 | A-9 | A-1 |
| 502 | R-8 | A-22 | A-9 | A-9 |
| 503 | R-8 | A-22 | A-9 | A-16 |
| 504 | R-8 | A-22 | A-9 | A-22 |
| 505 | R-8 | A-22 | A-16 | A-1 |
| 506 | R-8 | A-22 | A-16 | A-9 |
| 507 | R-8 | A-22 | A-16 | A-16 |
| 508 | R-8 | A-22 | A-16 | A-22 |
| 509 | R-8 | A-22 | A-22 | A-1 |
| 510 | R-8 | A-22 | A-22 | A-9 |
| 511 | R-8 | A-22 | A-22 | A-16 |
| 512 | R-8 | A-22 | A-22 | A-22 |

wobei die Gruppen A₁, A₂ und A₃ jeweils mit einem oder mehreren Resten R³ substituiert sein können.

Explizite Beispiele für Verbindungen der Formel (I) sind in der folgenden Tabelle gezeigt:

| | |
|---|---|
| 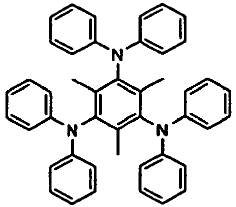 | 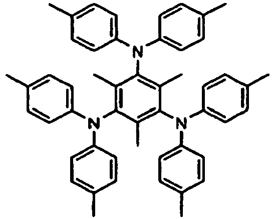 |
| 1 | 2 |
| 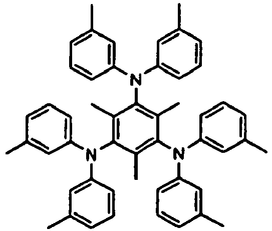 | 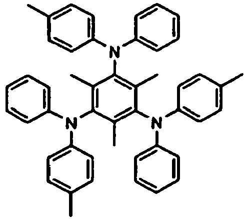 |
| 3 | 4 |
| 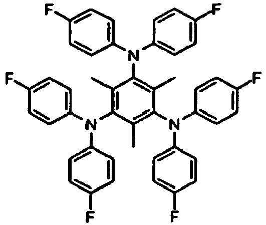 | 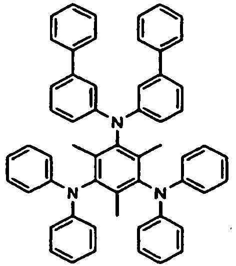 |
| 5 | 6 |
| 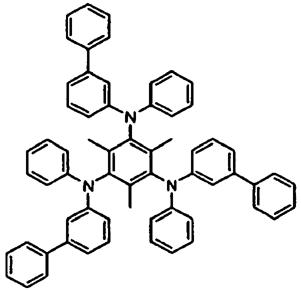 | 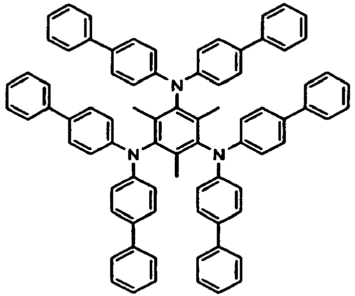 |
| 7 | 8 |
| 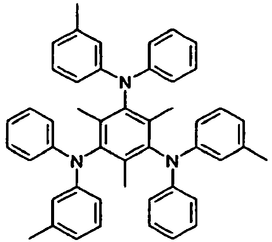 | 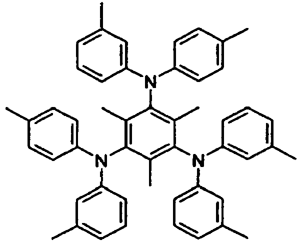 |
| 9 | 10 |
| 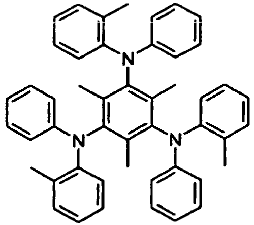 | 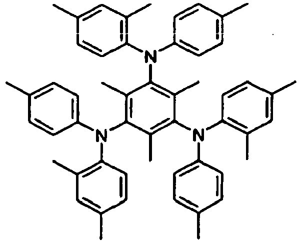 |
| 11 | 12 |
| 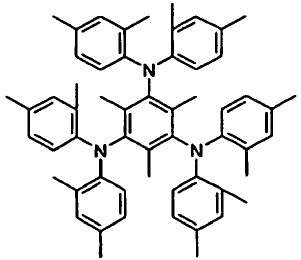 | 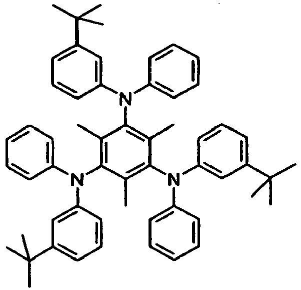 |
| 13 | 14 |
| 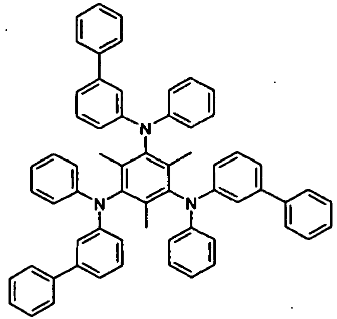 | 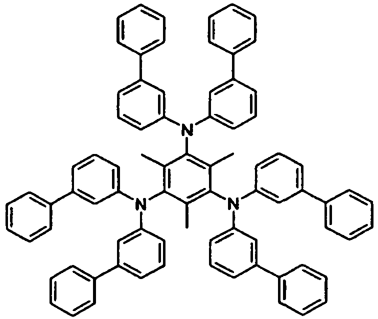 |
| 15 | 16 |
| 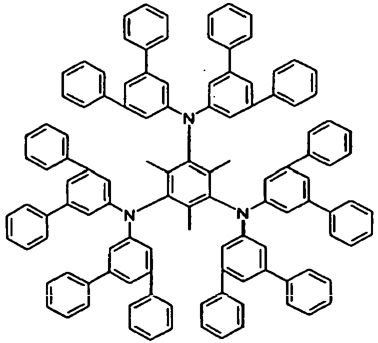 | 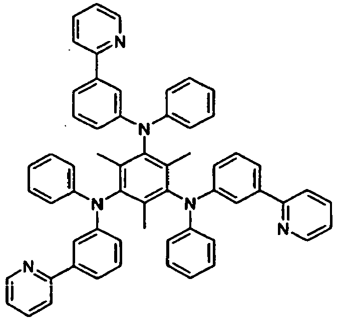 |
| 17 | 18 |
| 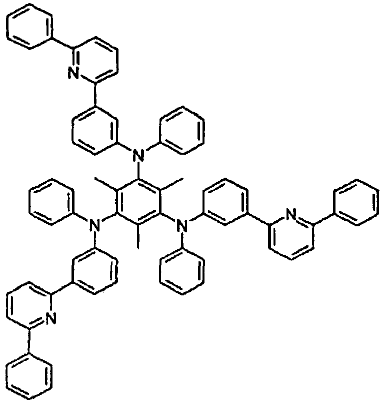 | 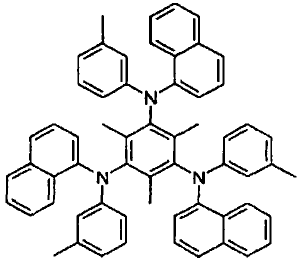 |
| 21 | 22 |
| 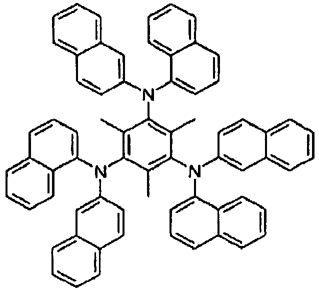 | |
| 23 | |
| 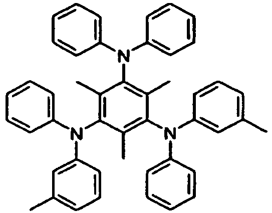 | 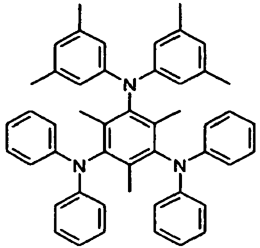 |
| 25 | 26 |
| 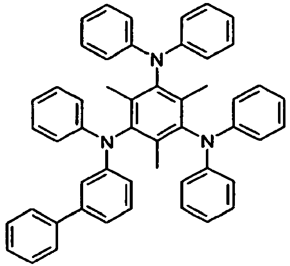 | 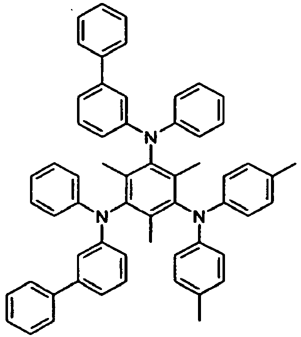 |
| 27 | 28 |
| 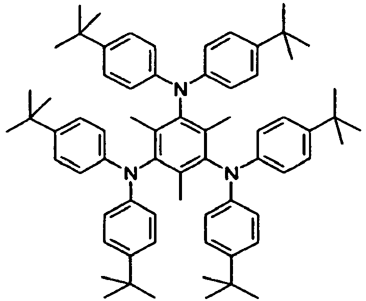 | 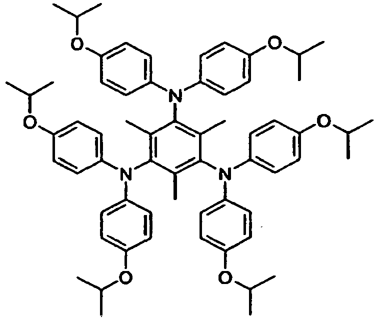 |
| 29 | 30 |
| 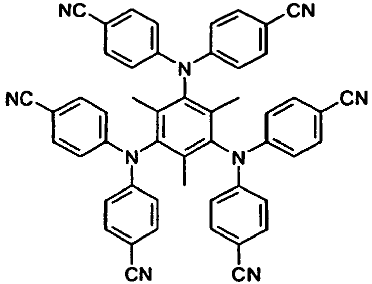 | 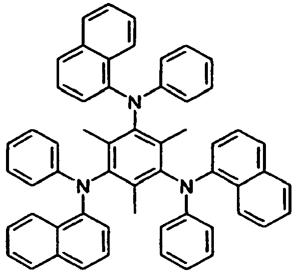 |
| 31 | 32 |
| 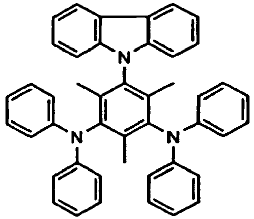 | 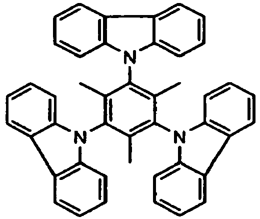 |
| 33 | 34 |
| 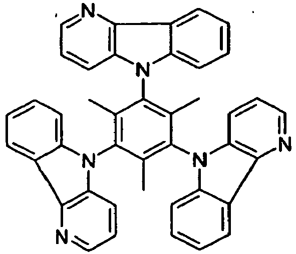 | 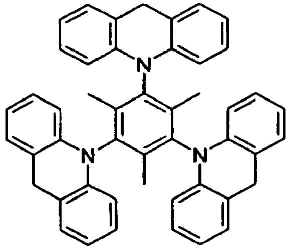 |
| 35 | 36 |
| 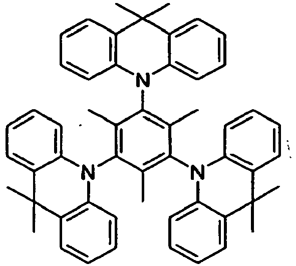 | 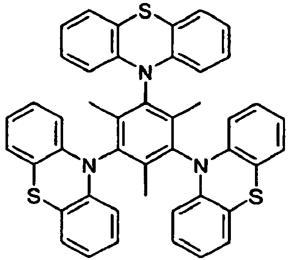 |
| 37 | 38 |
| 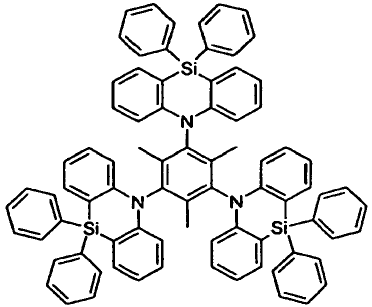 | 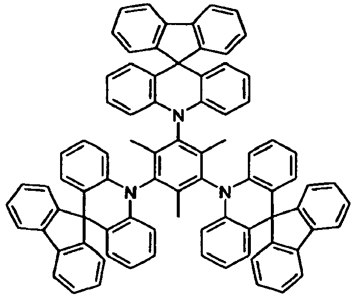 |
| 39 | 40 |
| 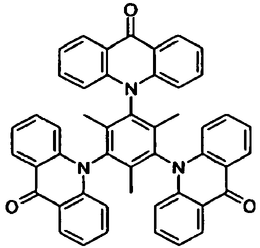 | 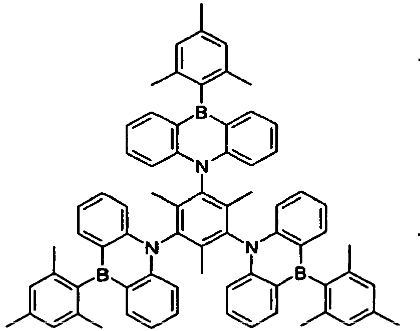 |
| 41 | 42 |
| 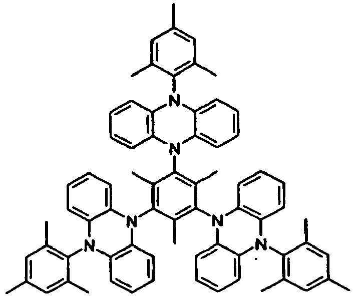 | 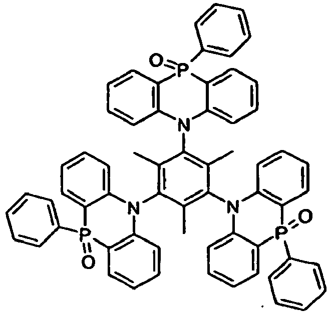 |
| 43 | 44 |
| 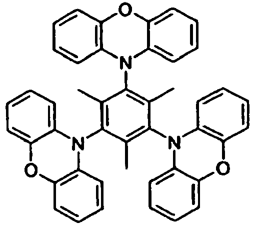 | 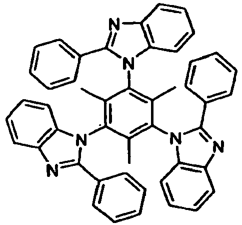 |
| 45 | 46 |
| 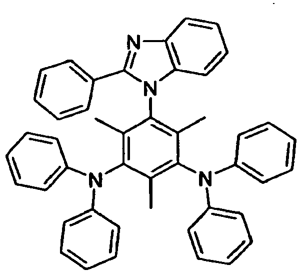 | 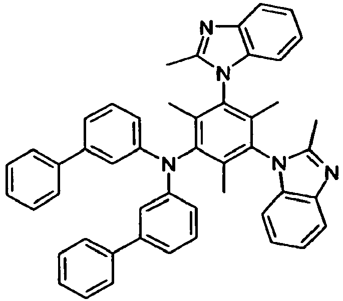 |
| 47 | 48 |
| 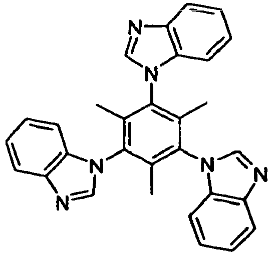 | 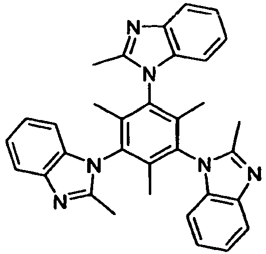 |
| 49 | 50 |
| 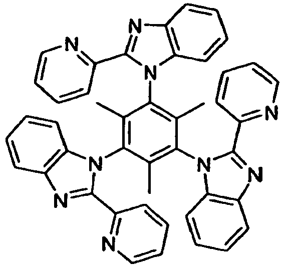 | 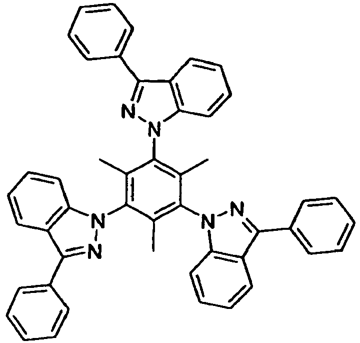 |
| 51 | 52 |
| 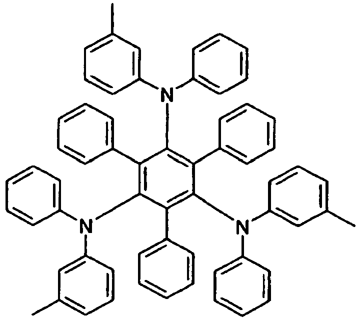 | 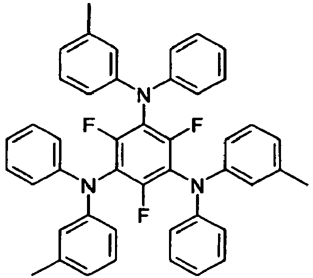 |
| 53 | 54 |
| 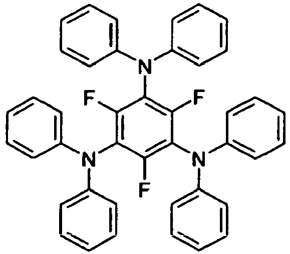 | 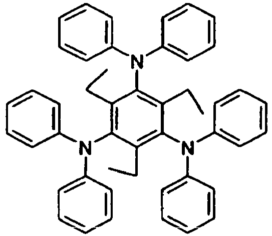 |
| 55 | 56 |
| 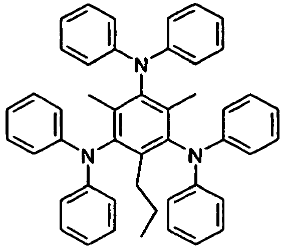 | 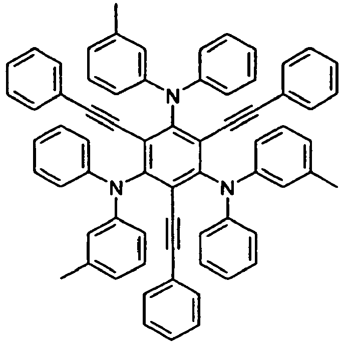 |
| 57 | 58 |
| 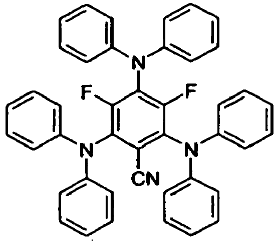 | 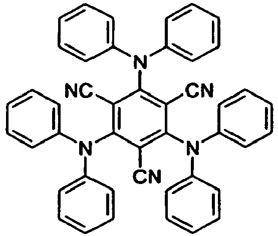 |
| 59 | 60 |

Die erfindungsgemäßen Verbindungen können durch bekannte organischchemische Syntheseverfahren hergestellt werden. Dazu zählen beispielsweise die Ullmann-Kupplung, die Hartwig-Buchwald-Kupplung, die Suzuki-Kupplung sowie Halogenierungsreaktionen.

Gemäß dem folgenden Schema 1 können die erfindungsgemäßen Verbindungen durch Pd-katalysierte Kupplung (Buchwald-Kupplung) oder Kupfer-katalysierte Kupplung (Ullmann-Kupplung) von 1,3,5-Tribromsubstituierten Benzolen, welche in den Positionen 2, 4 und 6 mit Resten R substituiert sind, mit aromatischen oder heteroaromatischen sekundären Aminen oder Heterocyclen mit NH-Funktion erhalten werden. Dabei kann die aus der Literatur bekannte Vielzahl von aromatischen oder heteroaromatischen sekundären Aminen oder Heterocyclen, die eine NH-Funktion aufweisen, wie z.B. Pyrrole, Indole, Pyrazole, Imidazole,

Benzimidazole, Carbazole, Azacarbazole, Phenoxazine, Phenothiazine, und weitere eingesetzt werden.

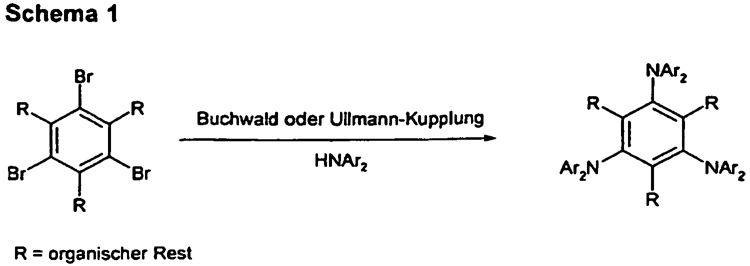

Die Aminierungsreaktion kann auch in zwei Stufen, wie in Schema 2 gezeigt, durchgeführt werden.

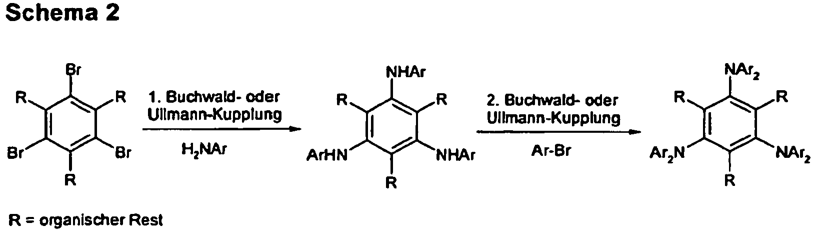

Durch den Einsatz von 1,3,5-Trihalogen-substituierten Benzolen, die unterschiedliche Halogene tragen, können bezüglich der Aminfunktionalität unsymmetrische erfindungsgemäße Verbindungen erhalten werden, indem sequenziell zwei Aminierungsreaktionen mit jeweils unterschiedlichen Aminen durchgeführt werden (s. Schema 3).

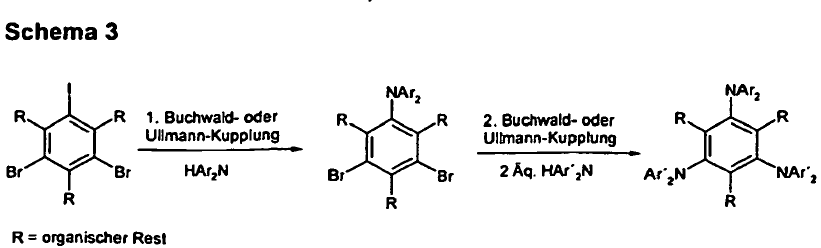

Als Edukte eignen sich hierfür beispielsweise:
- 1,3-Dibrom-5-iod-2,4,6-trimethylbenzol [1208325-27-2]
- 1,3-Dichlor-5-iod-2,4,6-trimethylbenzol [182119-63-7]
- 1-Brom-3,5-diiod-1,3,5-trimethylbenzol [124312-45-4]
- 1-Chlor-3,5-diiod-1,3,5-trimethylbenzol [41571-69-1]

Analog können auch andere Substituenten als Methyl in den Positionen 2, 4 und 6 anwesend sein.

Es ist weiterhin möglich, die aus den oben gezeigten Reaktionen erhaltenen Verbindungen weiter zu funktionalisieren. Schema 4 zeigt ein exemplarisches Beispiel hierfür. So kann die erfindungsgemäße Arylaminoverbindung mit NBS, bevorzugt in para-Position zum Stickstoff, bromiert werden. Bevorzugt werden dabei so viele Äquivalente NBS wie freie para-Positionen vorhanden sind verwendet. Die eingeführten Brom-Funktionen können dann in einem zweiten Schritt nach üblichen Methoden, z.B. in Aminierungs-Reaktionen, Suzuki-, Negishi-, Yamamoto-, Sonogashira-, Grignard-Cross-Kupplungen, Pd-katalysierten Boranylierungen, Veretherungen, Cyanierungen, Silylierungen, etc., um nur einige zu nennen, weiter umgesetzt werden. Dabei kann auch eine Umsetzung hin zu Oligomeren, Polymeren oder Dendrimeren erfolgen. In Schema 3 sind exemplarisch eine Aminierung nach Buchwald bzw. eine Arylkupplung nach Suzuki exemplarisch gezeigt.

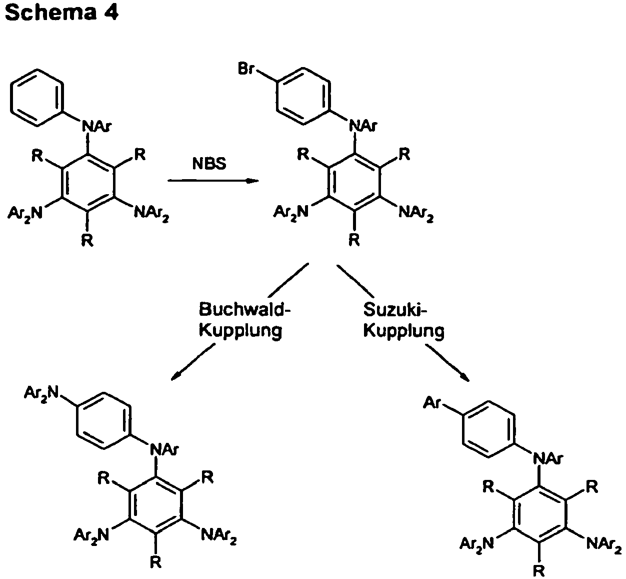

Gegenstand der Erfindung ist somit weiterhin ein Verfahren zur Herstellung einer Verbindung gemäß Formel (I), dadurch gekennzeichnet, dass mindestens eine Zwischenstufe einer Formel (Z) 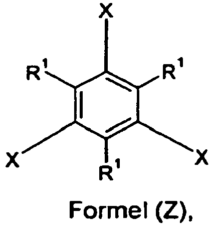 wobei R¹ definiert ist wie im Zusammenhang mit Formel (I), und wobei
- X: gleich oder verschieden eine beliebige reaktive Gruppe darstellt, beispielsweise Cl, Br, I, Boronsäure, Boronsäureester oder Sulfonsäureestergruppen,
mit mindestens einer Arylaminoverbindung oder mindestens einer heterocyclischen Verbindung enthaltend mindestens eine NH-Funktion umgesetzt wird.

Für die Verarbeitung der Verbindungen gemäß Formel (I) aus flüssiger Phase, beispielsweise durch Spin-Coating oder durch Druckverfahren, sind Formulierungen der Verbindungen erforderlich. Diese Formulierungen können beispielsweise Lösungen, Dispersionen oder Miniemulsionen sein.

Gegenstand der Erfindung ist daher weiterhin eine Formulierung, insbesondere eine Lösung, Dispersion oder Miniemulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in den Anmeldungen WO 2002/072714 und WO 2003/019694 und der darin zitierten Literatur beschrieben.

Die Verbindungen gemäß Formel (I) eignen sich für den Einsatz in elektronischen Vorrichtungen, insbesondere in organischen Elektrolumineszenzvorrichtungen (OLEDs). Die Verbindungen können, unter anderem abhängig von der Substitution, in unterschiedlichen Funktionen und/oder Schichten eingesetzt werden. Bevorzugt werden die Verbindungen als Lochtransportmaterialien in einer lochtransportierenden Schicht, als Matrixmaterialien in einer emittierenden Schicht, als Elektronenblockiermaterialien und als Excitonenblockiermaterialien eingesetzt.

Ein weiterer Gegenstand der Erfindung ist die Verwendung der Verbindungen gemäß Formel (I) in elektronischen Vorrichtungen. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt ausgewählt aus organischen Elektrolumineszenzvorrichtungen (OLEDs).

Gegenstand der Erfindung ist weiterhin eine elektronische Vorrichtung, enthaltend Anode, Kathode sowie mindestens eine organische Schicht, wobei die organische Schicht mindestens eine Verbindung der Formel (I) enthält. Dabei ist die elektronische Vorrichtung bevorzugt ausgewählt aus den oben genannten Vorrichtungen und besonders bevorzugt eine organische Elektrolumineszenzvorrichtung (OLED).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, *Multiphoton Organic EL Device Having Charge Generation Layer),* Auskopplungsschichten und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt Die in den jeweiligen Schichten und Funktionen bevorzugt eingesetzten Verbindungen werden in späteren Abschnitten explizit offenbart.

Es ist erfindungsgemäß bevorzugt, wenn die Verbindung gemäß Formel (I) in einer elektronischen Vorrichtung enthaltend einen oder mehrere phosphoreszierende Dotanden eingesetzt wird. Dabei kann die Verbindung in unterschiedlichen Schichten, bevorzugt in einer Elektronentransportschicht, einer Lochtransportschicht, einer Lochinjektionsschicht oder in der emittierenden Schicht verwendet werden. Die Verbindung gemäß Formel (I) kann aber auch erfindungsgemäß in einer elektronischen Vorrichtung, enthaltend einen oder mehrere fluoreszierende Dotanden und keine phosphoreszierenden Dotanden, eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen. Weitere Beispiele für geeignete phosphoreszierende Dotanden können der in einem späteren Abschnitt folgenden Tabelle entnommen werden.

In einer bevorzugten Ausführungsform der vorliegenden Erfindung werden die Verbindungen der Formel (I) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%. Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vo).-% an der Gesamtmischung.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 20061117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, Indenocarbazolderivate, z. B. gemäß WO 2010/136109 und WO 2011/000455, oder verbrückte Carbazole, z. B gemäß WO 2011/088877 und WO 2011/128017.

Wenn die Verbindung gemäß Formel (I) als Komponente eines Mixed-Matrix-Systems eingesetzt wird, wird sie bevorzugt in Kombination mit einem aromatischen Keton oder einem aromatischen Phosphinoxid, beispielsweise gemäß WO 2004/013080, WO 2004/093207, WO 2006/005627 oder WO 2010/006680, oder in Kombination mit einem Triazinderivat, beispielsweise gemäß WO 2010/015306, WO 2007/063754 oder WO 2008/056746, oder in Kombination mit einem Carbazolderivat, Indolocarbazolderivat oder Indenocarbazolderivat, z. B. gemäß WO 2007/063754, WO 2008/056746, WO 2010/136109 oder WO 2011/000455 eingesetzt.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

In einer weiteren bevorzugten Ausführungsform der Erfindung werden die Verbindungen gemäß Formel (I) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt.

Wird die Verbindung gemäß Formel (I) als Lochtransportmaterial in einer Lochtransportschicht eingesetzt, so kann die Verbindung als Reinmaterial, d.h. in einem Anteil von 100 % in der Lochtransportschicht eingesetzt werden, oder sie kann in Kombination mit weiteren Verbindungen in der Lochtransportschicht eingesetzt werden.

In einer weiteren Ausführungsform der Erfindung werden die die Verbindungen gemäß Formel (I) als fluoreszierende Dotanden in einer emittierenden Schicht eingesetzt. Die Verbindungen sind insbesondere dann zur Verwendung als fluoreszierende Dotanden geeignet, wenn sie mit einem oder mehreren aromatischen Systemen, bevorzugt aromatischen Systemen enthaltend 12 bis 20 aromatische Ringatome, substituiert sind. Die erfindungsgemäßen Verbindungen werden bevorzugt als grüne oder blaue Emitter verwendet.

Der Anteil der Verbindung gemäß Formel (I) als Dotand in der Mischung der emittierenden Schicht beträgt in diesem Fall zwischen 0.1 und 50.0 Vol.-%, bevorzugt zwischen 0.5 und 20.0 Vol.-%, besonders bevorzugt zwischen 0.5 und 8.0 Vol.-%. Entsprechend beträgt der Anteil des Matrixmaterials zwischen 50.0 und 99.9 Vol.-%, bevorzugt zwischen 80.0 und 99.5 Vol.-%, besonders bevorzugt zwischen 92.0 und 99.5 Vol.-%.

Bevorzugte Matrixmaterialien zur Verwendung in Kombination mit den erfindungsgemäßen Verbindungen als fluoreszierende Dotanden sind in einem der folgenden Abschnitte aufgeführt. Sie entsprechen den als bevorzugt aufgeführten Matrixmaterialien für fluoreszierende Dotanden.

In einer weiteren bevorzugten Ausführungsform der Erfindung werden die Verbindungen gemäß Formel (I) als Elektronenblockiermaterial eingesetzt. Die Verbindungen werden in diesem Fall bevorzugt in einer Elektronenblockierschicht eingesetzt, welche sich zwischen Anode und emittierender Schicht befindet, bevorzugt zwischen Lochtransportschicht und emittierender Schicht.

In einer weiteren bevorzugten Ausführungsform der Erfindung werden die Verbindungen gemäß Formel (I) als Excitonenblockiermaterial eingesetzt. Die Verbindungen werden in diesem Fall bevorzugt in einer Excitonenblockierschicht eingesetzt, welche sich zwischen Anode und emittierender Schicht befindet, bevorzugt zwischen Lochtransportschicht und emittierender Schicht. Wenn die Verbindungen gemäß Formel (I) als Excitonenblockiermaterial eingesetzt werden, weisen diese als Gruppen A bevorzugt Gruppen der Formel (II) auf, wobei Ar¹ eine Phenylgruppe oder eine ortho- oder meta-Biphenylgruppe darstellt, weiche jeweils mit einem oder mehreren Resten R² substituiert sein kann.

Die erfindungsgemäße organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren, wobei die verschiedenen Farben in dieser Ausführungsform der Erfindung zusammen weißes Licht ergeben. Insbesondere bevorzugt sind Dreischichtsysteme, also Systeme mit drei emittierenden Schichten, wobei eine oder mehrere dieser Schichten eine Verbindung gemäß Formel (I) enthalten kann und wobei die drei Schichten blaue, grüne und orangefarbene oder rote Emission zeigen (für den prinzipiellen Aufbau siehe z. B. WO 2005/011013). Ebenso eignen sich in solchen Systemen für weiße Emission Emitter, welche breitbandige Emissionsbanden aufweisen und dadurch weiße Emission zeigen. Alternativ und/oder zusätzlich können die erfindungsgemäßen Verbindungen in solchen Systemen auch in einer Lochtransportschicht oder Elektronentransportschicht oder in einer anderen Schicht vorhanden sein.

Im Folgenden werden die in den erfindungsgemäßen elektronischen Vorrichtungen bevorzugt eingesetzten weiteren Funktionsmaterialien aufgeführt.

Die in der folgenden Tabelle aufgeführten Verbindungen stellen besonders geeignete phosphoreszierende Dotanden dar.

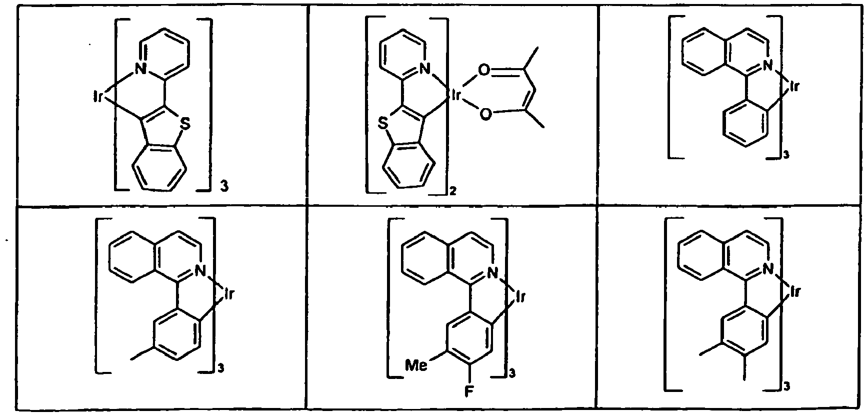

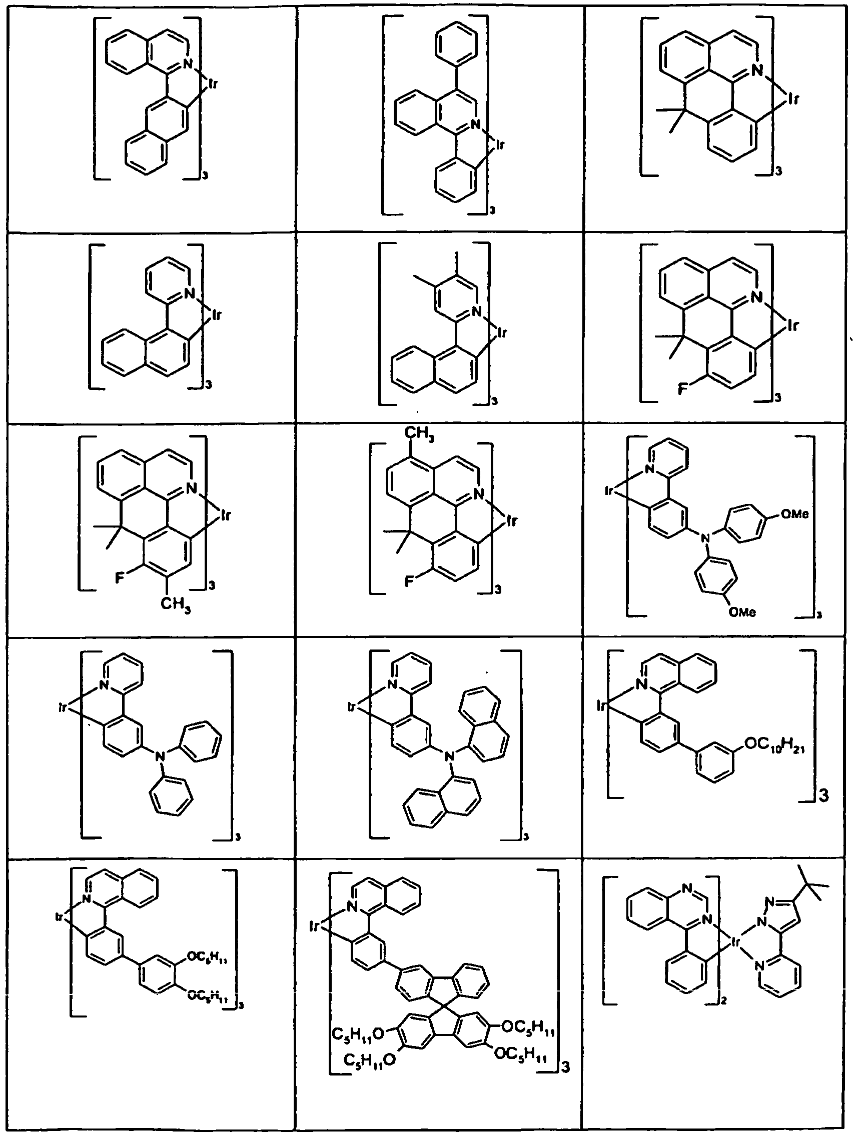

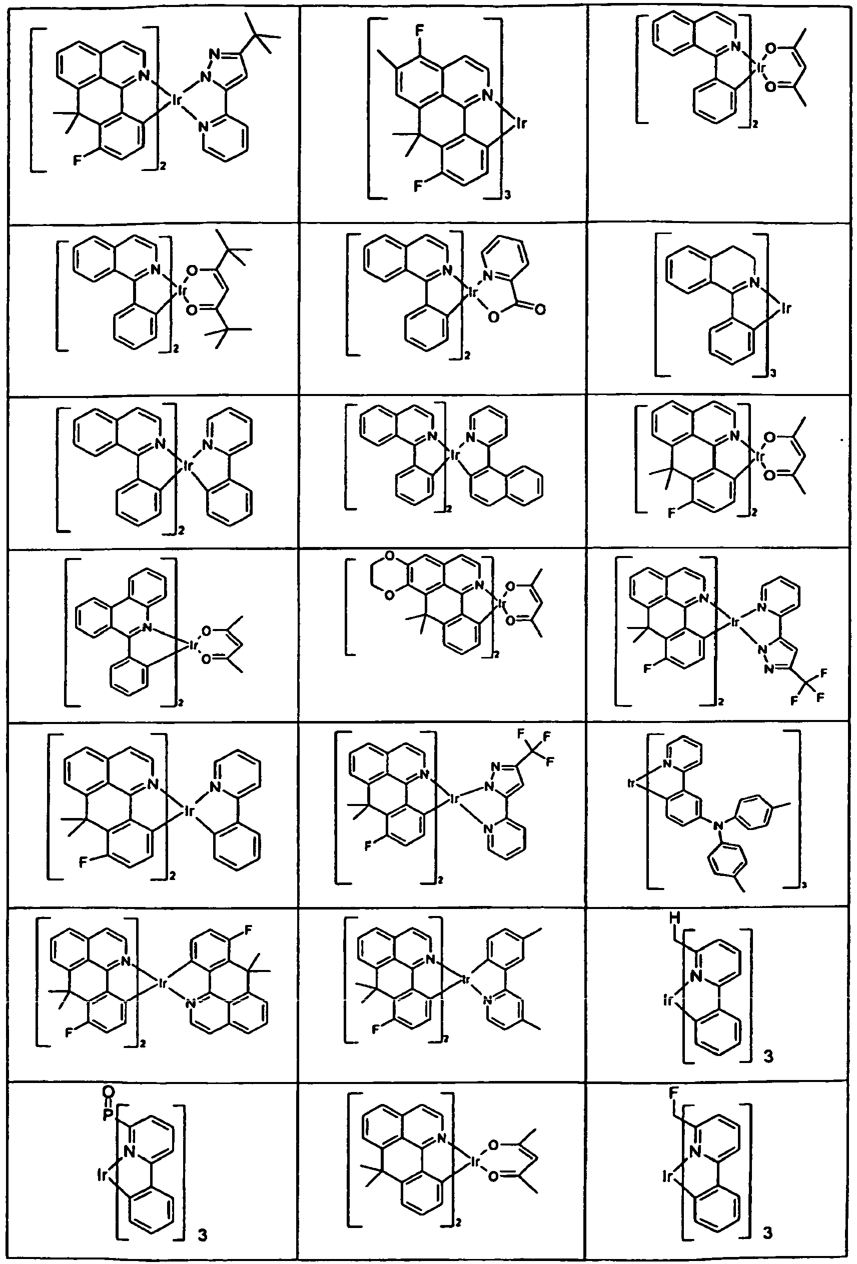

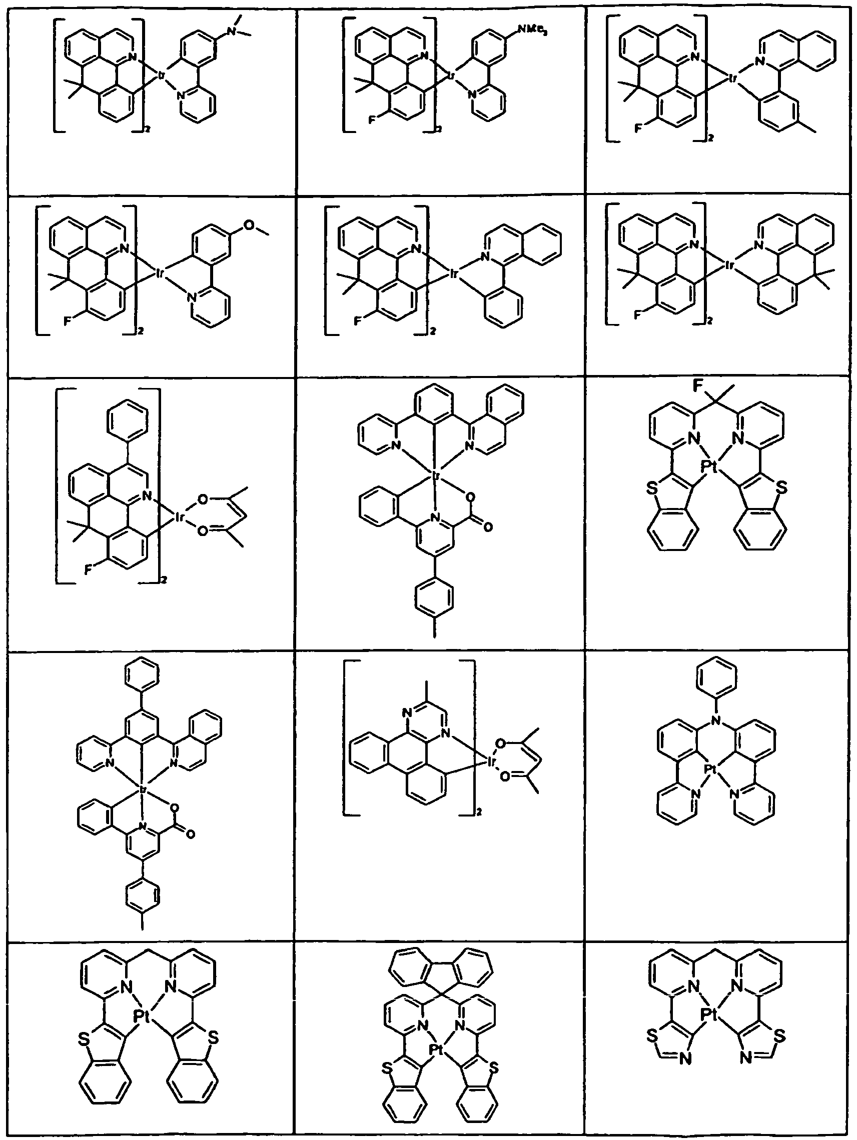

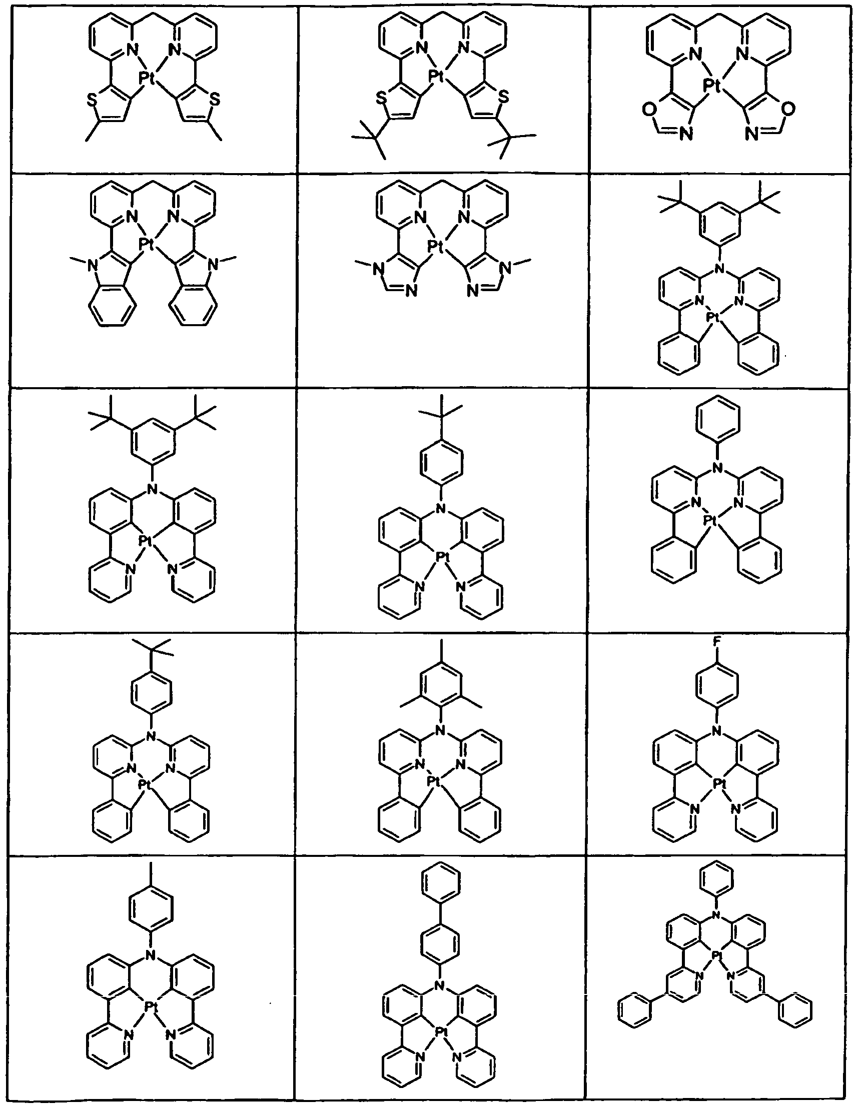

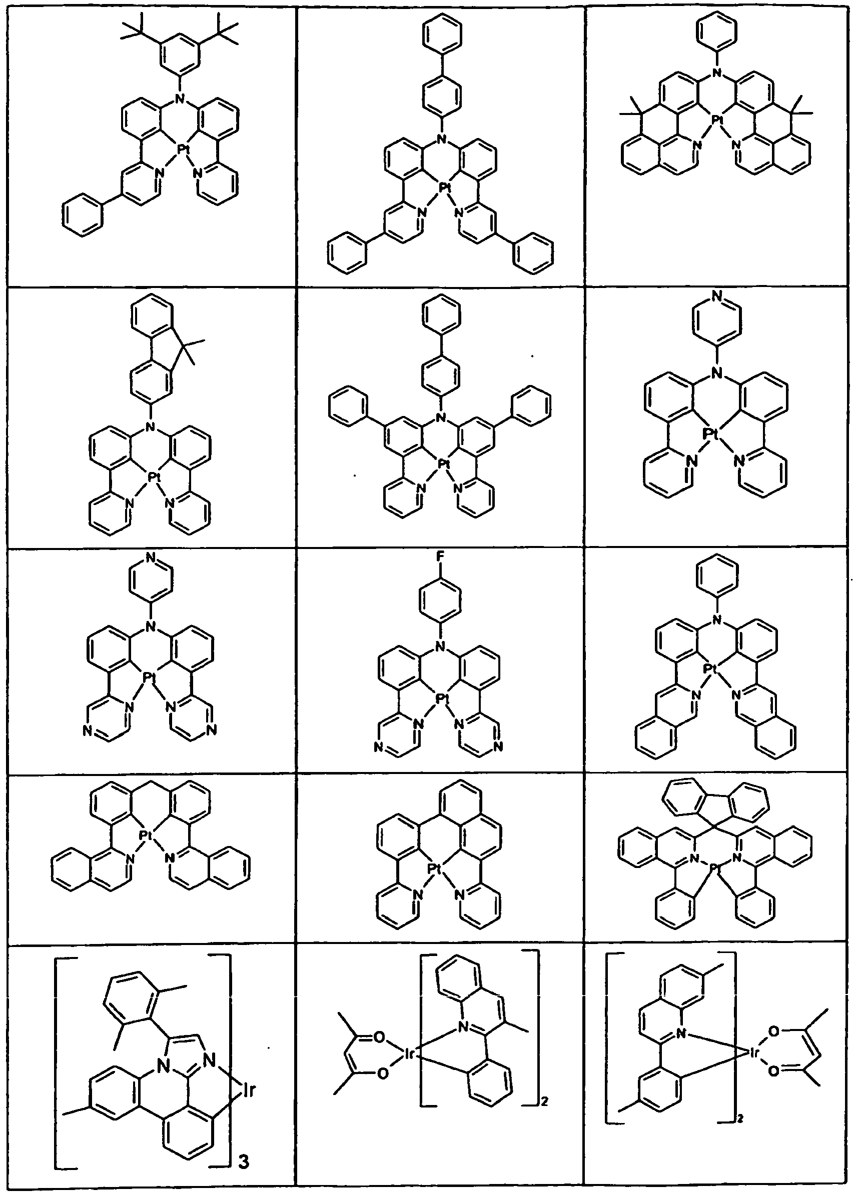

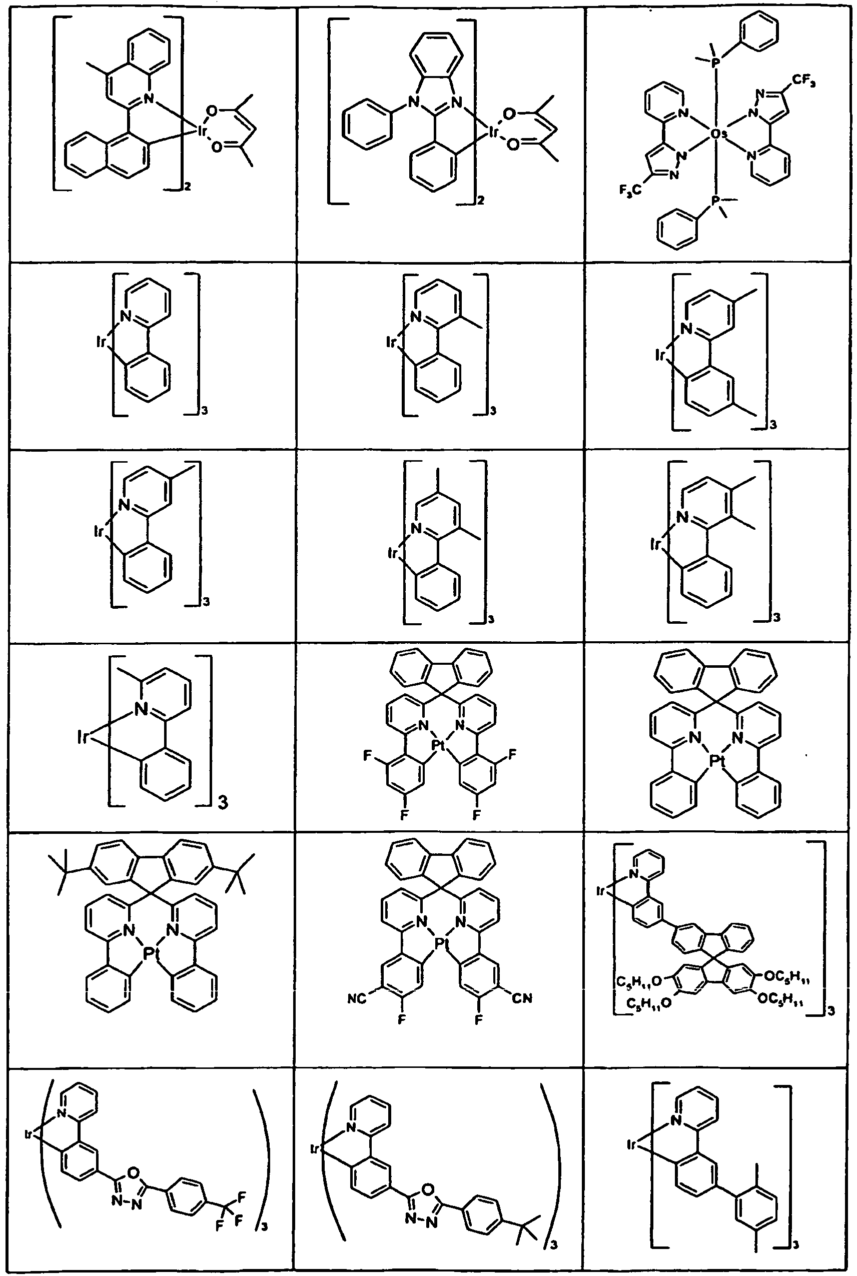

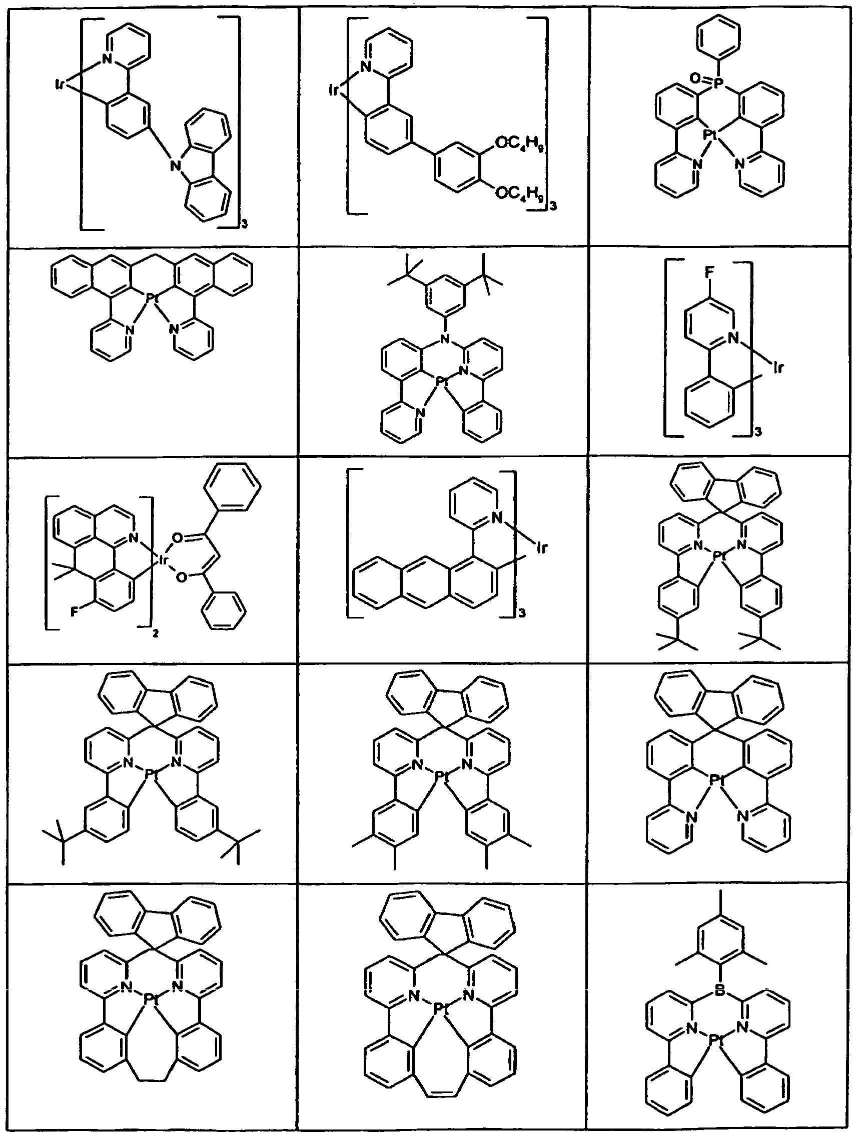

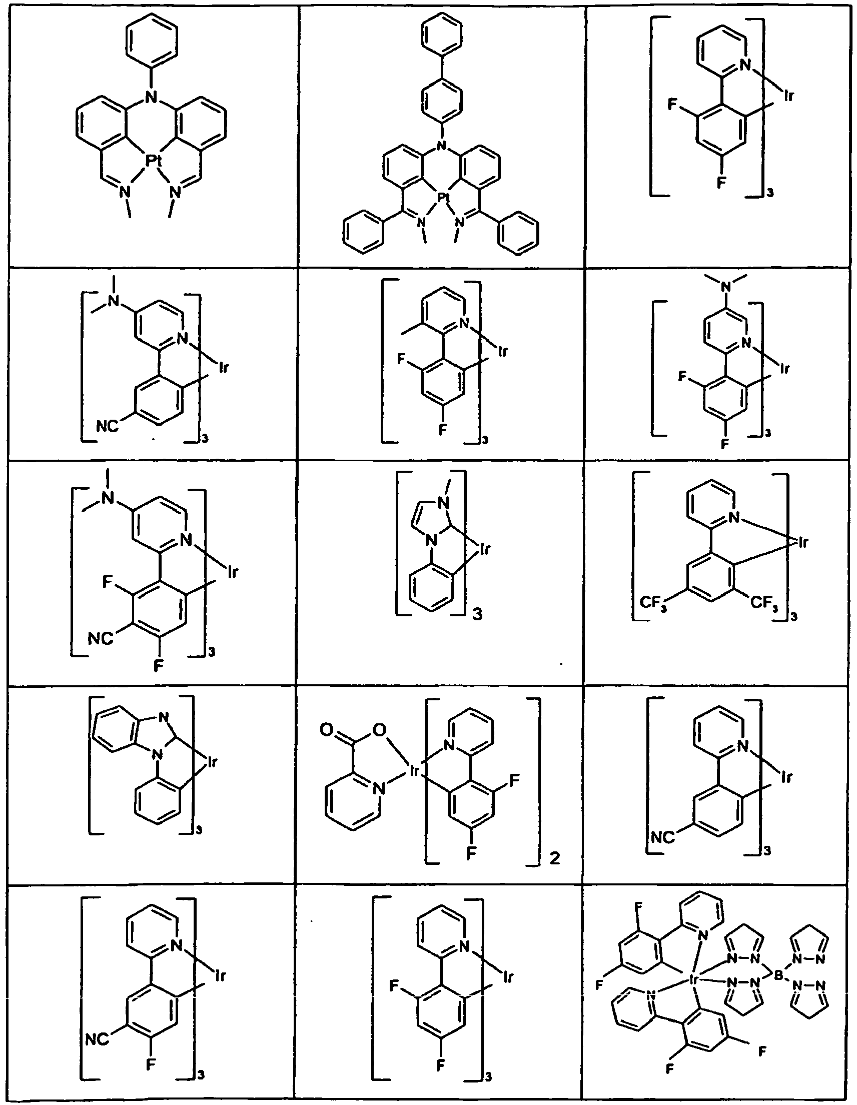

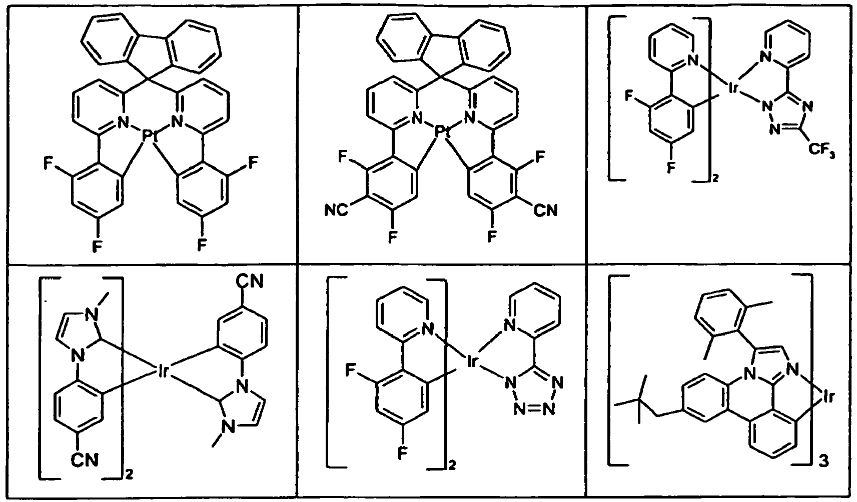

Bevorzugte fluoreszierende Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Styrylphosphine und Styrylether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Beispiele für fluoreszierende Dotanden aus der Klasse der Styrylamine sind substituierte oder unsubstituierte Tristilbenamine oder die fluoreszierenden Dotanden, die in WO 2006/000388, WO 2006/058737, WO 2006/000389, WO 2007/065549 und WO 2007/115610 beschrieben sind. Weiterhin bevorzugt sind die in WO 2010/012328 offenbarten kondensierten Kohlenwasserstoffe. Weiterhin können auch die Verbindungen gemäß Formel (I) als fluoreszierende Dotanden verwendet werden.

Geeignete fluoreszierende Dotanden sind weiterhin die in JP 2006/001973, WO 2004/047499, WO 2006/098080, WO 2007/065678, US 200510260442 und WO 2004/09211 offenbarten Strukturen.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Geeignete Matrixmaterialien, bevorzugt für fluoreszierende Dotanden, sind beispielsweise die in WO 2004/018587, WO 2008/006449, US 5935721, US 2005/0181232, JP 2000/273056, EP 681019, US 2004/0247937 und US 2005/0211958 offenbarten Strukturen.

Bevorzugte Matrixmaterialien für phosphoreszierende Dotanden sind neben den erfindungsgemäßen Verbindungen Carbazolderivate (z. B. CBP (N,N-Biscarbazolylbiphenyl) oder Verbindungen gemäß WO 2005/039246, US 2005/0069729, JP 2004/288381, EP 1205527 oder WO 2008/086851), Triarylamine, Azacarbazole (z. B. gemäß EP 1617710, EP 1617711, EP 1731584, JP 2005/347160), indolocarbazolderivate, z. B. gemäß WO 2007/063754 oder WO 2008/056746, Ketone (z. B. gemäß WO 2004/093207 oder WO 2010/006680), Phosphinoxide, Sulfoxide und Sulfone (z. B. gemäß WO 2005/003253), Oligophenylene, aromatische Amine (z. B. gemäß US 2005/0069729), bipolare Matrixmaterialien (z. B. gemäß WO 2007/137725), Silane (z. B. gemäß WO 2005/111172), Azaborole oder Boronester, z. B. gemäß WO 2006/117052, Triazinderivate, z. B. gemäß WO 2010/015306, WO 2007/063754 oder WO 2008/056746, Zinkkomplexe (z. B. gemäß WO 2009/062578) Aluminiumkomplexe (z. B. BAlq), Diazasilol- und Tetraazasilol-Derivate, z. B. gemäß WO 2010/054730, Indenocarbazolderivate, z. B. gemäß WO 2010/136109 und WO 2011/000455 oder Diazaphosphole, z. B. gemäß WO 2010/054730.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispiels-weise Ag, Pt oder Au. Es können andererseits auch Metall/Metalloxid-Elektroden (z. B. AUNi/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Die erfindungsgemäßen Verbindungen zeichnen sich insbesondere dadurch aus, dass sie bei Verwendung in organischen Elektrolumineszenzvorrichtungen gute Leistungseffizienzen, geringe Betriebsspannungen und lange Lebensdauern der Vorrichtungen bewirken.

Weiterhin sind die Verbindungen oxidationsstabil, temperaturstabil und weisen eine hohe Glasübergangstemperatur auf, was sowohl für die Prozessierbarkeit, beispielsweise aus Lösung oder aus der Gasphase, als auch für die Verwendung in elektronischen Vorrichtungen vorteilhaft ist.

Schließlich weisen die Verbindungen ein hochliegendes angeregtes Triplettniveau auf, was insbesondere bei Verwendung in einer emittierenden Schicht in Kombination mit einer phosphoreszierenden Emitterverbindung hoch erwünscht ist.

Die Erfindung wird durch die nachfolgenden Anwendungsbeispiele näher erläutert, wobei die Erfindung nicht auf den Umfang der Beispiele beschränkt ist.

### Ausführungsbeispiele

### A) Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden.

### Beispiel 1:

### 2,4,6-Trimethyl-N,N,N',N',N",N"-hexaphenyl-benzol-1,3,5-triamin

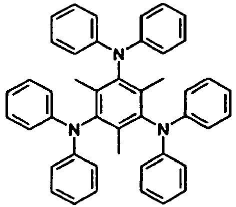

Eine Lösung von 85.0 g (238 mmol) 1,3,5-Tribrom-2,4,6-trimethylbenzol [608-72-0] und 161.2 g (953 mmol) Diphenylamin in 2000 ml Toluol wird konsekutiv mit 14.0 ml (14 mmol) Tris-tert-butylphosphin, 1M in Toluol, 1.6 g (7 mmol) Palladium(II)acetat und dann mit 103.0 g (1.1 mol) Natrium-tert-butylat versetzt. Man erhitzt die Reaktionsmischung 16 h unter Rückfluss, lässt auf 60 °C erkalten, gibt 20 ml Essigsäure und 250 ml Wasser zu, lässt auf Raumtemperatur erkalten, trennt die wässrige Phase ab, wäscht die org. Phase einmal mit 500 ml Wasser und einmal mit 500 ml ges. Kochsalzlösung und entfernt dann das Toluol im Vakuum. Der Rückstand wird einmal mit 1000 ml Ethanol ausgekocht, im Vakuum getrocknet und dann sechsmal aus DMF (ca. 5 ml/g) umkristallisiert. Anschließend erfolgt eine zweimalige fraktionierte Sublimation im Hochvakuum (p ca. 10⁻⁶ mbar, T ca. 290 °C). Ausbeute: 69.6 g (112 mmol), 47 % Reinheit n. HPLC > 99.9 %ig.

Analog wurden folgende Verbindungen erhalten, wobei anstelle von Diphenylamin die in der folgenden Tabelle aufgeführten Amine verwendet wurden:

| Bsp. | Amin | Produkt | Ausbeute |
|---|---|---|---|
| **2** | 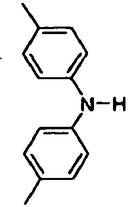 | 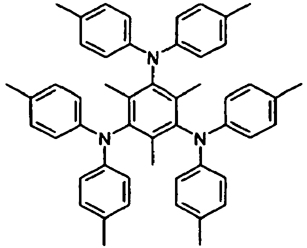 | 56% |
| | 620-93-9 | | |
| **3** | 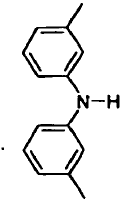 | 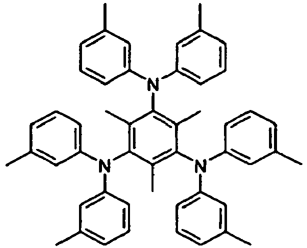 | 38 % |
| | 626-13-1 | | |
| **4** | 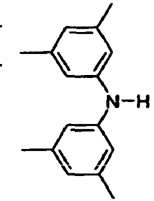 | 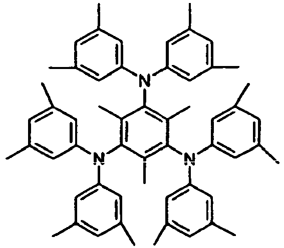 | 46% |
| | 5369-25-5 | | |
| **5** | 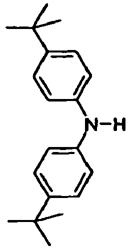 | 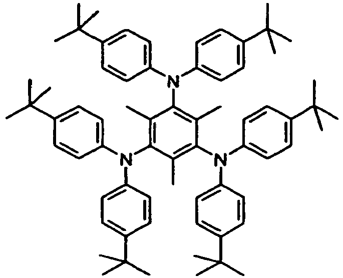 | 52 % |
| | 4627-22-9 | | |
| **6** | 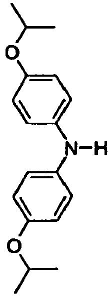 | 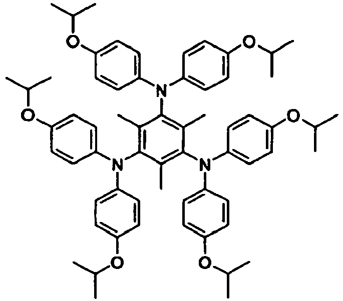 | 27 % |
| | 2204-93-5 | | |
| **7** | 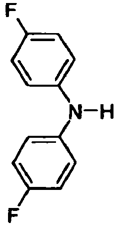 | 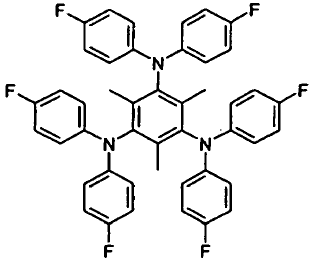 | 58 % |
| | 330-91-6 | | |
| **8** | 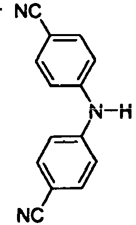 | 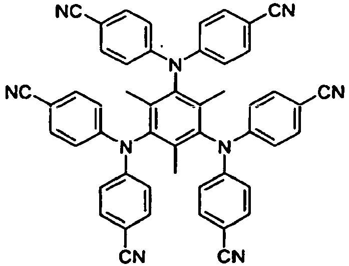 | 55 % |
| | 36602-05-8 | | |
| **9** | 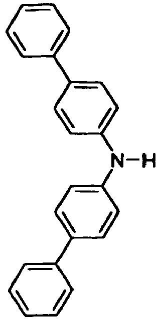 | 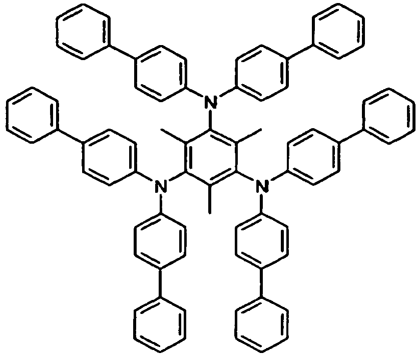 | 58 % |
| | 102113-98-4 | Produkt wurde nicht sublimiert, sondern bei 280 °C im Vakuum getempert | |
| **10** | 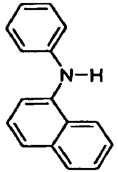 | 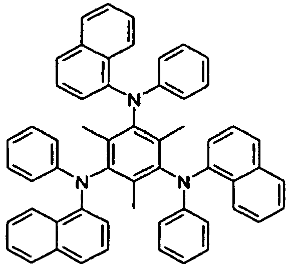 | 47 % |
| | 90-30-2 | | |
| **11** | 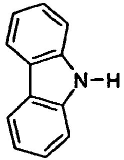 | 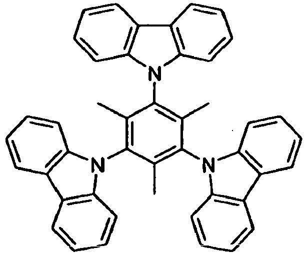 | 50 % |
| | 86-74-8 | | |
| **12** | 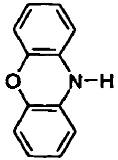 | 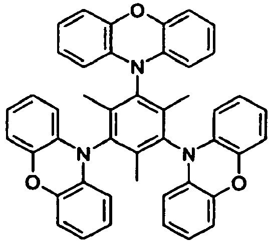 | 50 % |
| | 135-67-1 | | |
| **13** | 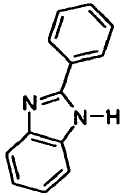 | 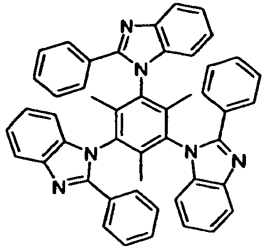 | 65 % |
| | 716-79-0 | | |
| **19** | 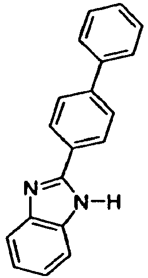 | 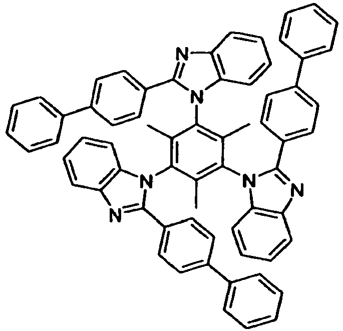 | 57 % |
| | 2562-77-8 | | |
| **20** | 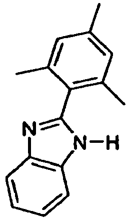 | 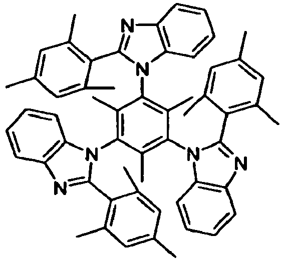 | 11 % |
| | 36677-31-3 | | |
| **21** | 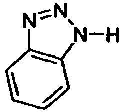 | 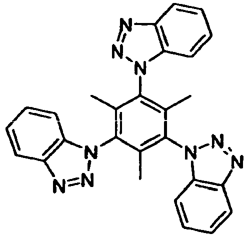 | 28 % |
| | 95-15-7 | | |

Analog wurden folgende Verbindungen erhalten, wobei anstelle von 1,3,5-Tribrom-2,4,6-trimethylbenzol die in der folgenden Tabelle aufgeführten Tribrombenzolderivate verwendet wurden:

| Bsp. | Tribromid | Produkt | Ausbeute |
|---|---|---|---|
| **14** | 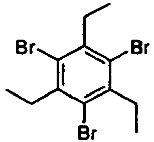 | 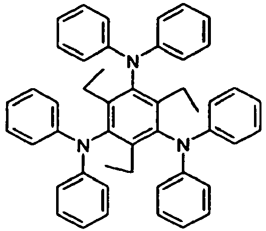 | 46 % |
| | 80717-52-8 | | |
| **15** | 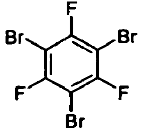 | 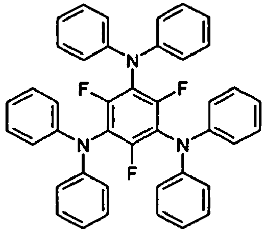 | 52 % |
| | 2368-49-2 | | |
| **16** | 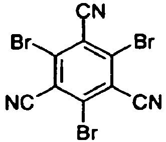 | 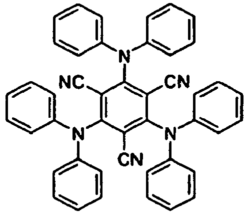 | 41 % |
| | 60510-14-7 | | |
| **17** | 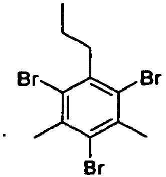 | 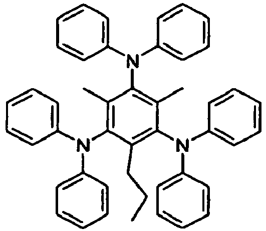 | 34 % |
| | 859785-26-5 | | |
| **18** | 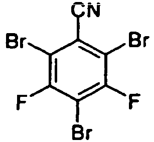 | 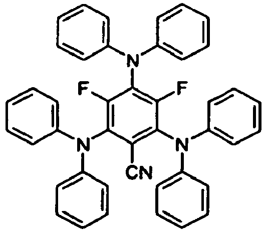 | 37 % |
| | 943528-40-3 | | |

### B) Devicebeispiele: Herstellung und Charakterisierung von organischen Elektrolumineszenzvorrichtungen

Erfindungsgemäße Elektrolumineszenzvorrichtungen können, wie beispielsweise in WO 2005/003253 allgemein beschrieben, dargestellt werden. Im Folgenden werden die Ergebnisse unterschiedlich aufgebauter erfindungsgemäßer OLEDs gegenübergestellt. Der grundlegende Aufbau, die verwendeten Materialien, der Dotierungsgrad und ihre Schichtdicken sind zur besseren Vergleichbarkeit identisch.

Es werden OLEDs mit den erfindungsgemäßen Verbindungen nach Beispiel **1, 2, 3, 4, 5, 10, 11, 13, 14, 19, 20** und **21** als Lochtransportmaterial (HTL2), bzw. als Matrixmaterial in einem Mixed-Matrix-System, bzw. als Elektronentransportmaterial in folgendem Schichtaufbau hergestellt (Device-Beispiele **D1-D15**):

| | |
|---|---|
| Lochinjektionsschicht (HIL) | 70 nm 2,2',7,7'-Tetrakis(di-para-tolyl-amino)spiro-9,9'-bifluoren |
| Lochtransportschicht (HTL1) | 5 nm 1,4,5,8,9,11-Hexaazatriphenylenhexacarbonitril |
| Lochtransportschicht (HTL2) | 15 nm, siehe Tabelle erfindungsgemäße Verbindung, |
| Emissionsschicht (EML): Host und Dotand | 40 nm siehe Tabelle 1, Anteile in Vol.-% |
| Elektronenleiter (ETL) | 40 nm ETM1 (50 Vol.-%) bzw. siehe Tabelle erfindungsgemäße Verbindung und ETM2 (50 Vol.-%) |
| Kathode | 1 nm ETM2, darauf 100 nm Al. |

Weiterhin werden zum Vergleich OLEDs enthaltend das Lochtransportmaterial HTL3 gemäß dem Stand der Technik hergestellt (Device-Beispiele **V1-V2**).

Die Strukturen der verwendeten Verbindungen mit Ausnahme der erfindungsgemäßen Verbindungen (s. Tabelle Synthesebeispiele) sind der Übersichtlichkeit halber im Folgenden abgebildet.

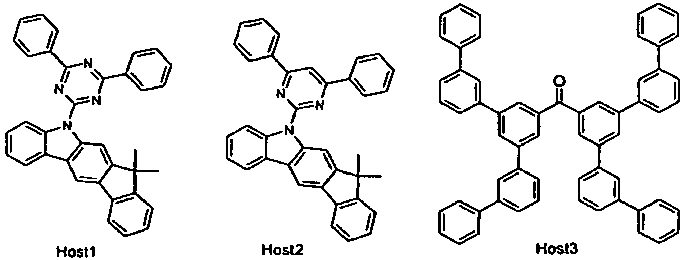

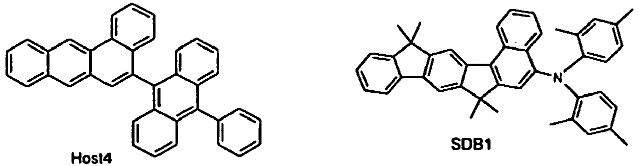

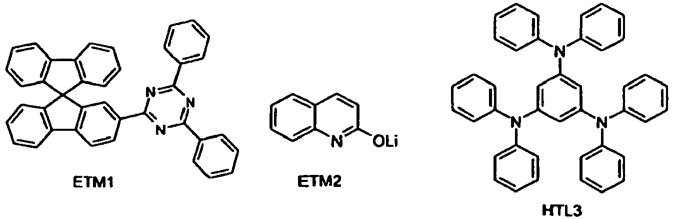

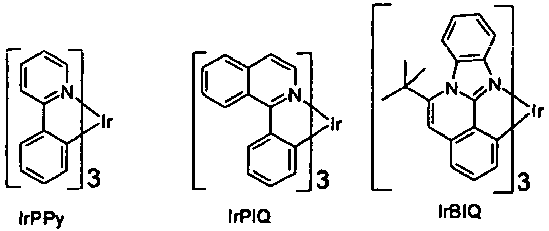

Zur Charakterisierung der hergestellten OLEDs werden die Elektrolumineszenzspektren sowie die externe Quanteneffizienz (EQE, gemessen in %) in Abhängigkeit von der Helligkeit, berechnet aus Strom-Spannungs-Helligkeit-Kennlinien (IUL-Kennlinien), bestimmt.

| Tabelle 1: Device-Ergebnisse | | | | | | |
|---|---|---|---|---|---|---|
| **Bsp.** | **HTL2** | **Host / Dotand** | **ETL** | **EQE bei 1000 cd/m² [%]** | **Spannung bei 1000 cd/m² [V]** | **CIE x / y** |
| **D1** | Bsp.1 | Host 1, 90 % | ETM1, 50 % | 15.8 | 8.8 | 0.35 / |
| | | IrPPy, 10 % | ETM2, 50 % | | | 0.62 |
| **D2** | Bsp.2 | Host 1.90 % | ETM1, 50 % | 16.0 | 4.3 | 0.35 / |
| | | IrPPy, 10 % | ETM2, 50 % | | | 0.62 |
| **D3** | Bsp.4 | Host 1, 90 % | ETM1, 50 % | 16.3 | 4.0 | 0.35 / |
| | | IrPPy, 10 % | ETM2, 50 % | | | 0.62 |
| **D4** | Bsp.10 | Host 1, 90 % | ETM1, 50 % | 14.2 | 3.8 | 0.35 / |
| | | IrPPy, 10 % | ETM2, 50 % | | | 0.62 |
| **D5** | Bsp.14 | Host 1, 90 % | ETM1, 50 % | 13.5 | 8.5 | 0.36 / |
| | | IrPPy, 10 % | ETM2, 50 % | | | 0.63 |
| **D6** | Bsp.10 | Host 1, 85 % | ETM1, 50 % | 12.1 | 3.4 | 0.68 / |
| | | IrPIQ, 15 % | ETM2, 50 % | | | 0.31 |
| **D7** | Bsp.4 | Host 2. 85 % | ETM1, 50 % | 12.3 | 6.2 | 0.16 / |
| | | IrBIQ, 15 % | ETM2, 50 % | | | 0.29 |
| **D8** | Bsp.4 | Host 3, 65 % | ETM1, 50 % | 13.6 | 5.7 | 0.16 / |
| | | Bsp.1, 25 % | ETM2, 50 % | | | 0.29 |
| | | IrBIQ, 10 % | | | | |
| **D9** | Bsp.2 | Host 1, 70 % | ETM1, 50 % | 17.3 | 3.6 | 0.35 / |
| | | Bsp.2, 20 % | ETM2, 50 % | | | 0.62 |
| | | IrPPy, 10 % | | | | |
| **D10** | Bsp.2 | Host 1, 70 % | ETM1, 50 % | 15.2 | 4.6 | 0.35 / |
| | | Bsp. 11, 20 % | ETM2, 50 % | | | 0.62 |
| | | IrPPy, 10 % | | | | |
| **D11** | Bsp.4 | Host 4, 90 % | ETM1, 50 % | 8.1 | 5.2 | 0.14 / |
| | | SDB1, 10 % | ETM2, 50 % | | | 0.14 |
| **D12** | Bsp.3 | Host 4, 90 % | Bsp. 13, 50 % | 7.9 | 5.2 | 0.14 / |
| | | SDB1, 10 % | ETM2, 50 % | | | 0.14 |
| **D13** | Bsp.5 | Host 4, 90 % | Bsp.19, 50 % | 8.0 | 5.5 | 0.14 / |
| | | SDB1, 10 % | ETM2, 50 % | | | 0.13 |
| **D14** | Bsp.1 | Host 1, 65% | Bsp.20, 50 % | 16.8 | 4.4 | 0.35 / |
| | | Bsp.11, 20 % | ETM2, 50 % | | | 0.63 |
| | | IrPPy, 15 % | | | | |
| | | | | | | |
| **D15** | Bsp.1 | Host 2, 70 % | Bsp.21, 50 % | 17.0 | 4.5 | 0.35 / |
| | | Bp.11, 20% | ETM2, 50 % | | | 0.62 |
| | | IrPPy, 10 % | | | | |
| **V1** | HTL3 | Host 2, 85 % | ETM1, 50 % | 5.7 | 6.3 | 0.16 / |
| | Vgl.-Bsp. | IrBIQ, 15 % | ETM2, 50 % | | | 0.31 |
| **V2** | HTL3 | Host 1, 90 % | ETM1, 50 % | 11.0 | 4.5 | 0.36 / |
| | Vgl.-bsp. | IrPPy, 10 % | ETM2, 50 % | | | 0.62 |

Anhand der gemessenen Leistungsdaten für die Device-Beispiele **D1-D15** wird deutlich, dass mit den Verbindungen gemäß der vorliegenden Erfindung sowohl bei Verwendung als Lochtransportmaterialien (**D1-D11**) als auch bei Verwendung als Matrixmaterialien für phosphoreszierende Emitter (**D8-D10**) als auch bei Verwendung als Elektronentransportmaterial (**D12-15**) sehr gute Ergebnisse bezüglich Betriebsspannung und Leistungseffizienz erhalten werden können. Die Beispiele **D1-D10, D14, D15** betreffen dabei OLEDs mit phosphoreszierender Emitterschicht, und die Beispiele **D11-D13** betreffen OLEDs mit fluoreszierender Emitterschicht.

Der Vergleich mit dem Lochtransportmaterial HTL3 gemäß dem Stand der Technik (Device-Beispiele **V30** und **V31**) zeigt, dass bei identischem Aufbau mit den erfindungsgemäßen Verbindungen bessere Leistungsdaten erzielt werden (Vgl. **V1** mit erfindungsgemäßem Beispiel **D7** sowie **V2** mit erfindungsgemäßen Beispielen **D1-D5**).

## Patentansprüche

1. Verbindung einer Formel (I) 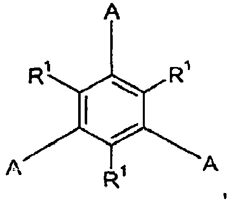 wobei A bei jedem Auftreten gleich oder verschieden eine Gruppe der folgenden Formel (II) oder (III) darstellt 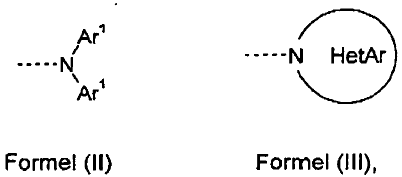 wobei die gestrichelte Linie ausgehend vom Stickstoffatom die Bindung der Gruppe A an den zentralen Benzolring darstellt;
wobei die Gruppe der Formel (III) ausgewählt ist aus den Formeln (III-1) bis (III-3) 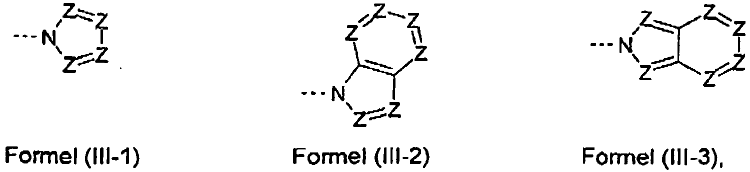 wobei
Z bei jedem Auftreten gleich oder verschieden CR² oder N darstellt;
wobei die Gruppen Ar¹ in einer Gruppe A der Formel (II) bei jedem Auftreten gleich oder verschieden eine Arylgruppe mit 6 bis 10 aromatischen Ringatomen darstellen, die mit einem oder mehreren Resten R² substituiert sein kann; und wobei die beiden Gruppen Ar¹ über eine Gruppe Y verbunden sein können, so dass mit dem Stickstoffatom der Gruppe A ein Ring gebildet wird, wobei
Y ausgewählt Ist aus einer Einfachbindung, BR², C(R²)₂, Si(R²)₂, NR², PR², P(=O)R², P(=S)R², O, S, S=O und S(=O)₂;
und wobei weiterhin gilt:
R¹ ist bei jedem Auftreten gleich oder verschieden F, Cl, Br, I, B(OR³)₂, CHO, C(=O)R³, CR³=C(R³)₂, CN, C(=O)OR³, C(=O)N(R³)₂, Si(R³)₃, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-,-S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 60 aromatischen Ringatomen, das jeweils durch einen oder mehrere Reste R³ substituiert sein kann, oder ein heteroaromatisches Ringsystem mit 5 bis 60 aromatischen Ringatomen, welches nicht über ein Ring-Stickstoffatom gebunden ist, und das durch einen oder mehrere Reste R³ substitutiert sein kann, oder eine Aryloxy- oder Heteroaryloxygruppe mit 5 bis 60 aromatischen Ringatomen, die durch einen oder mehrere Reste R³ substituiert sein kann, oder eine Aralkyl- oder Heteroaralkylgruppe mit 5 bis 60 aromatischen Ringatomen, die durch einen oder mehrere Reste R³ substituiert sein kann;
R² ist bei jedem Auftreten gleich oder verschieden H, D, F, Cl, Br, I, B(OR³)₂, CHO, C(=O)R³, CR³=C(R³)_{2.} CN, C(=O)OR³, C(-O)N(R³)₂, Si(R³)₃, N(R³)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-,-S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, oder eine Aralkyl- oder Heteroaralkylgruppe mit 5 bis 60 aromatischen Ringatomen, die durch einen oder mehrere Reste R³ substituiert sein kann, wobei zwei oder mehr Reste R² miteinander verknüpft sein können und einen Ring bilden können;
R³ ist bei jedem Auftreten gleich oder verschieden H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, NR⁴, P(=O)(R⁴), -O-,-S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, oder eine Aralkyl- oder Heteroaralkylgruppe mit 5 bis 60 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden.

2. Verbindung nach Anspruch 1, **dadurch gekennzeichnet, dass** die Gruppe der Formel (II) eine Gruppe der folgenden Formeln (II-1) bis (II-11) darstellt 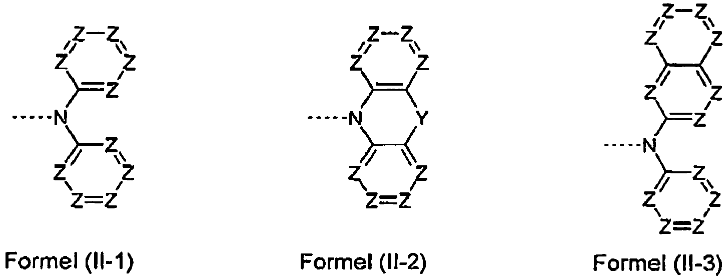 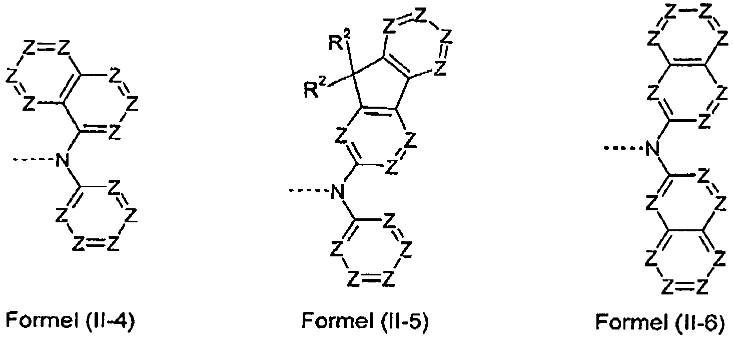 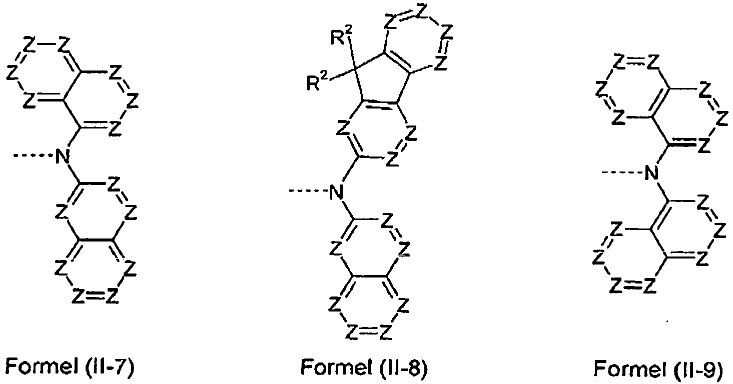 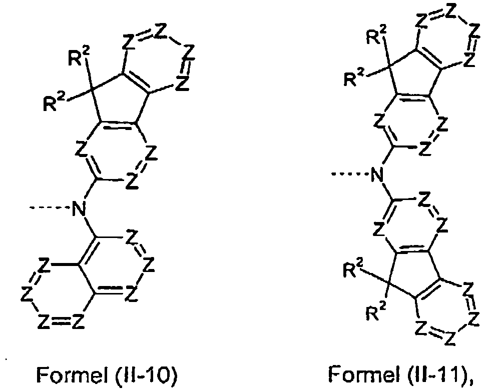 wobei R² wie in Anspruch 1 definiert ist,
Z bei jedem Auftreten gleich oder verschieden CR² oder N darstellt; und
Y ausgewählt ist aus einer Einfachbindung, BR², C(R²)₂, Si(R²)₂, NR², PR², P(=O)R², O, S, S=O und S(=O)₂.

3. Verbindung nach Anspruch 1 oder 2, **dadurch gekennzeichnet, dass** die beiden Gruppen Ar¹, welche in einer Gruppe der Formel (II) an ein gemeinsames N-Atom binden, gleich gewählt sind.

4. Verbindung nach einem oder mehreren der Ansprüche 1 bis 3,
**dadurch gekennzeichnet, dass** Y ausgewählt ist aus einer Einfachbindung, C(R²)₂, NR², O und S.

5. Verbindung nach einem oder mehreren der Ansprüche 1 bis 4,
**dadurch gekennzeichnet, dass** die Gruppe R¹ bei jedem Auftreten gleich oder verschieden ausgewählt ist aus F, CN, Si(R³)₃, oder einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, oder einer Alkenyl- oder Alkinylgruppe mit 2 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -C≡C-,-R³C=CR³-, Si(R³)₂, C=O, C=NR³, -NR³-, -O-, -S-, -C(=O)O-oder -C(=O)NR³- ersetzt sein können, oder einem aromatischen Ringsystem mit 5 bis 20 aromatischen Ringatomen, das jeweils mit einem oder mehreren Resten R³ substituiert sein kann, oder einem heteroaromatischen Ringsystem, das nicht über ein Ring-Stickstoffatom gebunden ist, und das jeweils mit einem oder mehreren Resten R³ substituiert ist.

6. Verbindung nach einem oder mehreren der Ansprüche 1 bis 5, **dadurch gekennzeichnet, dass** die Reste R¹ in Formel (I) gleich gewählt sind.

7. Verfahren zur Herstellung einer Verbindung nach einem oder mehreren der Ansprüche 1 bis 6, **dadurch gekennzeichnet, dass** mindestens eine Zwischenstufe einer Formel (Z) 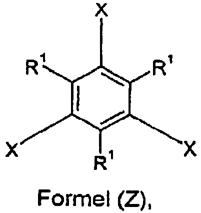 wobei R¹ definiert ist wie im Zusammenhang mit Formel (I) in Anspruch 1; und wobei
X gleich oder verschieden eine beliebige reaktive Gruppe darstellt, beispielsweise Cl, Br, I, Boronsäure, Boronsäureester oder Sulfonsäureestergruppen, mit mindestens einer Arylaminoverbindung oder mindestens einer heterocyclischen Verbindung enthaltend mindestens eine NH-Funktion umgesetzt wird.

8. Formulierung enthaltend mindestens eine Verbindung gemäß Formel (I) nach einem oder mehreren der Ansprüche 1 bis 6 sowie mindestens ein Lösungsmittel.

9. Elektronische Vorrichtung, enthaltend mindestens eine Verbindung gemäß einem oder mehreren der Ansprüche 1 bis 6.

10. Elektronische Vorrichtung gemäß Anspruch 9, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

11. Organische Elektrolumineszenzvorrichtung gemäß Anspruch 10, **dadurch gekennzeichnet, dass** die Verbindung gemäß einem oder mehreren der Ansprüche 1 bis 6 als Lochtransportmaterial in einer lochtransportierenden Schicht, als Matrixmaterial in einer emittierenden Schicht, als Elektronenblockiermaterial oder als Excitonenblockiermaterial vorhanden ist.

12. Organische Elektrolumineszenzvorrichtung gemäß Anspruch 10 oder 1, **dadurch gekennzeichnet, dass** die Verbindung gemäß einem oder mehreren der Ansprüche 1 bis 6 als Matrixmaterial in einer phosphoreszierenden emittierenden Schicht vorhanden ist, wobei zusätzlich zu der Verbindung gemäß einem oder mehreren der Ansprüche 1 bis 6 mindestens ein weiteres Matrixmaterial in der phosphoreszierenden emittierenden Schicht vorhanden ist.

## Claims

1. Compound of a formula (I) 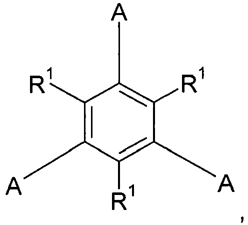 where A on each occurrence, identically or differently, represents a group of the following formula (II) or (III) 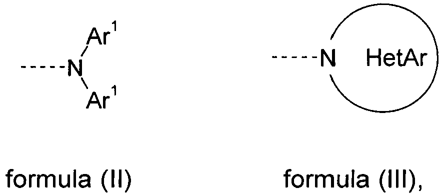 where the dashed line emanating from the nitrogen atom represents the bond from the group A to the central benzene ring;
where the group of the formula (III) is selected from the formulae (III-1) to (III-3) 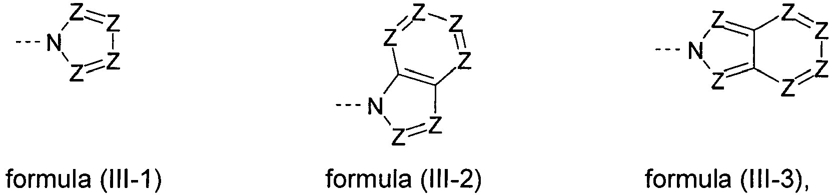 where
Z on each occurrence, identically or differently, represents CR² or N.
where the groups Ar¹ in a group A of the formula (II) on each occurrence, identically or differently, represent an aryl group having 6 to 10 aromatic ring atoms, which may be substituted by one or more radicals R², and where the two groups Ar¹ may be connected via a group Y so that a ring is formed with the nitrogen atom of the group A, where
Y is selected from a single bond, BR², C(R²)₂, Si(R²)₂, NR², PR², P(=O)R², P(=S)R², O, S, S=O and S(=O)₂;
and where furthermore:
R¹ is on each occurrence, identically or differently, F, Cl, Br, I, B(OR³)₂, CHO, C(=O)R³, CR³=C(R³)₂, CN, C(=O)OR³, C(=O)N(R³)₂, Si(R³)₃, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic ring system having 6 to 60 aromatic ring atoms, which may in each case be substituted by one or more radicals R³, or a heteroaromatic ring system having 5 to 60 aromatic ring atoms which is not bonded via a ring nitrogen atom, and which may be substituted by one or more radicals R³, or an aryloxy or heteroaryloxy group having 5 to 60 aromatic ring atoms, which may be substituted by one or more radicals R³, or an aralkyl or heteroaralkyl group having 5 to 60 aromatic ring atoms, which may be substituted by one or more radicals R³;
R² is on each occurrence, identically or differently, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, or an aralkyl or heteroaralkyl group having 5 to 60 aromatic ring atoms, which may be substituted by one or more radicals R³, where two or more radicals R² may be linked to one another and may form a ring;
R³ is on each occurrence, identically or differently, H, D, F, Cl, Br, I, B(OR⁴)₂, CHO, C(=O)R⁴, CR⁴=C(R⁴)₂, CN, C(=O)OR⁴, C(=O)N(R⁴)₂, Si(R⁴)³, N(R⁴)₂, NO₂, P(=O)(R⁴)₂, OSO₂R⁴, OR⁴, S(=O)R⁴, S(=O)₂R⁴, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C≡C-, Si(R⁴)₂, C=O, C=S, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, I, CN or NO₂, or an aromatic or heteroaromatic ring system having 5 to 60 aromatic ring atoms, which may in each case be substituted by one or more radicals R⁴, or an aryloxy or heteroaryloxy group having 5 to 60 aromatic ring atoms, which may be substituted by one or more radicals R⁴, or an aralkyl or heteroaralkyl group having 5 to 60 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring.

2. Compound according to Claim 1, **characterised in that** the group of the formula (II) represents a group of the following formulae (II-1) to (II-11) 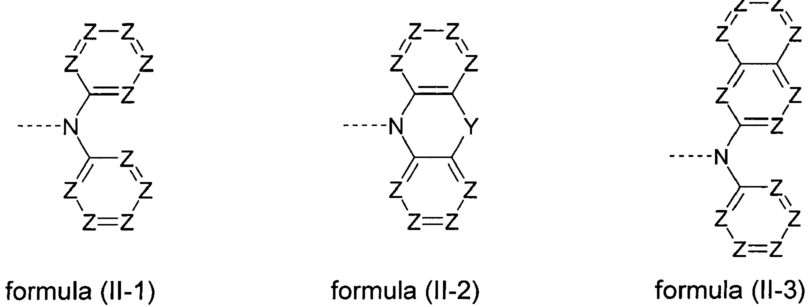 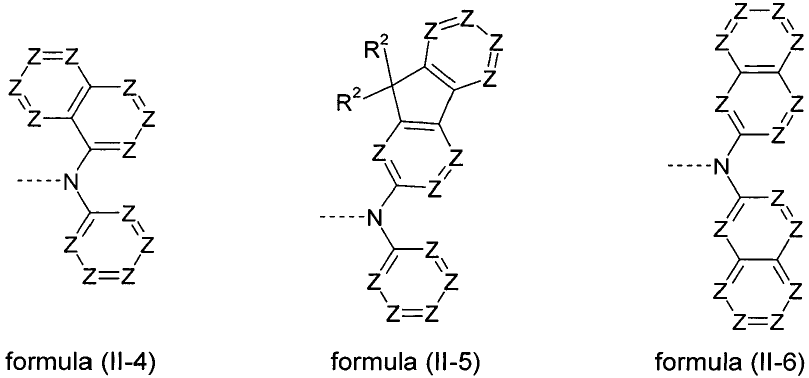 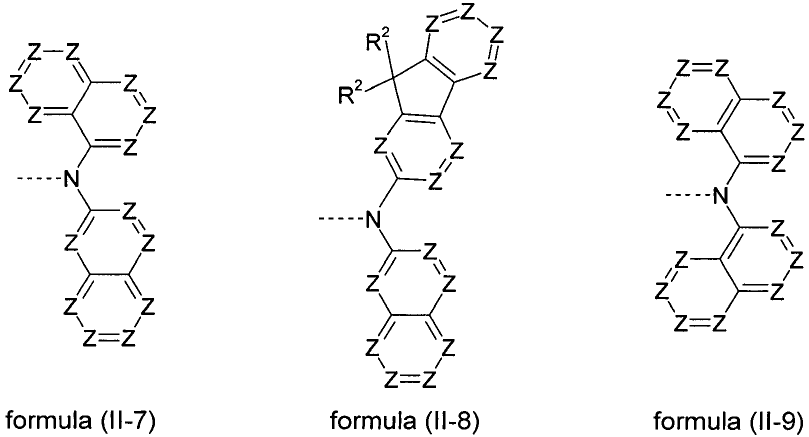 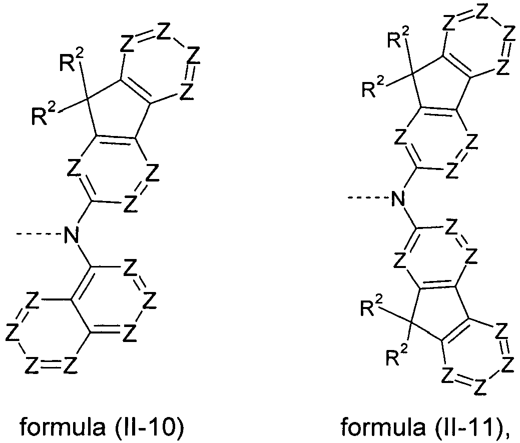 where R² is defined as in Claim 1,
Z on each occurrence, identically or differently, represents CR² or N; and
Y is selected from a single bond, BR², C(R²)₂, Si(R²)₂, NR², PR², P(=O)R², O, S, S=O and S(=O)₂.

3. Compound according to Claim 1 or 2, **characterised in that** the two groups Ar¹ which are bonded to a common N atom in a group of the formula (II) are selected identically.

4. Compound according to one or more of Claims 1 to 3, **characterised in that** Y is selected from a single bond, C(R²)₂, NR², O and S.

5. Compound according to one or more of Claims 1 to 4, **characterised in that** the group R¹ is selected on each occurrence, identically or differently, from F, CN, Si(R³)₃, or a straight-chain alkyl or alkoxy group having 1 to 10 C atoms or a branched or cyclic alkyl or alkoxy group having 3 to 10 C atoms, or an alkenyl or alkynyl group having 2 to 10 C atoms, where the above-mentioned groups may each be substituted by one or more radicals R³ and where one or more CH₂ groups in the above-mentioned groups may be replaced by -C≡C-, -R³C=CR³-, Si(R³)₂, C=O, C=NR³, -NR³-, -O-, -S-, -C(=O)O- or -C(=O)NR³-, or an aromatic ring system having 5 to 20 aromatic ring atoms, which may in each case be substituted by one or more radicals R³, or a heteroaromatic ring system which is not bonded via a ring nitrogen atom, and which is in each case substituted by one or more radicals R³.

6. Compound according to one or more of Claims 1 to 5, **characterised in that** the radicals R¹ in formula (I) are selected identically.

7. Process for the preparation of a compound according to one or more of Claims 1 to 6, **characterised in that** at least one intermediate of a formula (Z) 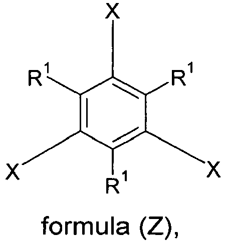 where R¹ is defined as in connection with formula (I) in Claim 1, and where
X represents, identically or differently, any desired reactive group, for example Cl, Br, I, boronic acid, boronic acid ester or sulfonic acid ester groups,
is reacted with at least one arylamino compound or at least one heterocyclic compound containing at least one NH function.

8. Formulation comprising at least one compound of the formula (I) according to one or more of Claims 1 to 6 and at least one solvent.

9. Electronic device containing at least one compound according to one or more of Claims 1 to 6.

10. Electronic device according to Claim 9, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

11. Organic electroluminescent device according to Claim 10, **characterised in that** the compound according to one or more of Claims 1 to 6 is present as hole-transport material in a hole-transporting layer, as matrix material in an emitting layer, as electron-blocking material or as exciton-blocking material.

12. Organic electroluminescent device according to Claim 10 or 11, **characterised in that** the compound according to one or more of Claims 1 to 6 is present as matrix material in a phosphorescent emitting layer, where, in addition to the compound according to one or more of Claims 1 to 6, at least one further matrix material is present in the phosphorescent emitting layer.

## Revendications

1. Composé de la formule (I) : 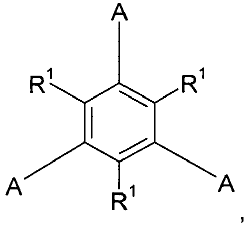 dans laquelle A représente, pour chaque occurrence, de manière identique ou différente, un groupe de la formule (II) ou (III) qui suit : 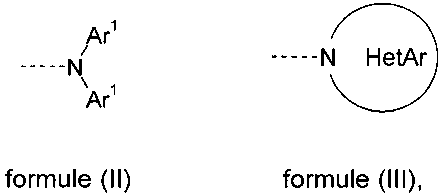 dans lesquelles la ligne en pointillés qui provient de l'atome d'azote représente la liaison depuis le groupe A jusqu'au cycle benzène central ;
où le groupe de la formule (III) est choisi parmi les formules (III-1) à (III-3) : 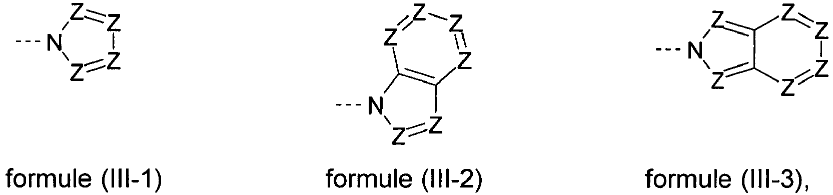 dans lesquelles :
Z représente, pour chaque occurrence, de manière identique ou différente, CR² ou N,
où les groupes Ar¹ dans un groupe A de la formule (II) représentent, pour chaque occurrence, de manière identique ou différente, un groupe aryle qui comporte 6 à 10 atomes de cycle aromatique, lequel peut être substitué par un radical ou plusieurs radicaux R², et où les deux groupes Ar¹ peuvent être connectés via un groupe Y de telle sorte qu'un cycle soit formé avec l'atome d'azote du groupe A, où
Y est choisi parmi une liaison simple, BR², C(R²)₂, Si(R²)₂, NR², PR², P(=O)R², P(=S)R², O, S, S=O et S(=O)₂ ;
et où en outre :
R¹ est, pour chaque occurrence, de manière identique ou différente, F, CI, Br, I, B(OR³)₂, CHO, C(=O)R³, CR³=C(R³)₂, CN, C(=O)OR³, C(=O)N(R³)₂, Si(R³)₃, NO₂, P(=O)(R³)₂, OSO₂R³, OR³, S(=O)R³, S(=O)₂R³, un groupe alkyle, alcoxy ou thio-alkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, où les groupes qui ont été mentionnés ci-avant peuvent chacun être substitués par un radical ou plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, CI, Br, I, CN ou NO₂, ou un système de cycle aromatique qui comporte 6 à 60 atomes de cycle aromatique, lequel peut dans chaque cas être substitué par un radical ou plusieurs radicaux R³, ou un système de cycle hétéroaromatique qui comporte 5 à 60 atomes de cycle aromatique, lequel n'est pas lié via un atome d'azote de cycle, et lequel peut être substitué par un radical ou plusieurs radicaux R³, ou un groupe aryloxy ou hétéroaryl-oxy qui comporte 5 à 60 atomes de cycle aromatique, lequel peut être substitué par un radical ou plusieurs radicaux R³, ou un groupe aralkyle ou hétéroaralkyle qui comporte 5 à 60 atomes de cycle aromatique, lequel peut être substitué par un radical ou plusieurs radicaux R³ ;
R² est, pour chaque occurrence, de manière identique ou différente, H, D, F, CI, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, où les groupes qui ont été mentionnés ci-avant peuvent chacun être substitués par un radical ou plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=S, C=NR³, -C(=O)O-, -C(=O)NR³-, NR³, P(=O)(R³), -O-, -S-, SO or SO₂ et où un ou plusieurs atome(s) de H dans les groupes qui ont été mentionnés ci-avant peut/peuvent être remplacé(s) par D, F, CI, Br, I, CN ou NO₂, ou un système de cycle aromatique ou hétéroaromatique qui comporte 5 à 60 atomes de cycle aromatique, lequel peut dans chaque cas être substitué par un radical ou plusieurs radicaux R³, ou un groupe aryloxy ou hétéroaryloxy qui comporte 5 à 60 atomes de cycle aromatique, lequel peut être substitué par un radical ou plusieurs radicaux R³, ou un groupe aralkyle ou hétéroaralkyle qui comporte 5 à 60 atomes de cycle aromatique, lequel peut être substitué par un radical ou plusieurs radicaux R³, où deux radicaux R² ou plus peuvent être liés l'un à l'autre ou les uns aux autres et peuvent former un cycle ;
R³ est, pour chaque occurrence, de manière identique ou différente, H, D, F, CI, Br, I, B(OR⁴)₂, CHO, C(=O)R⁴, CR⁴=C(R⁴)₂, CN, C(=O)OR⁴, C(=O)N(R⁴)₂, Si(R⁴)₃, N(R⁴)₂, NO₂, P(=O)(R⁴)₂, OSO₂R⁴, OR⁴, S(=O)R⁴, S(=O)₂R⁴, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, où les groupes qui ont été mentionnés ci-avant peuvent chacun être substitués par un radical ou plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=S, C=NR⁴, -C(=O)O-, -C(=O)NR⁴-, NR⁴, P(=O)(R⁴), -O-, -S-, SO ou SO₂ et où un ou plusieurs atome(s) de H dans les groupes qui ont été mentionnés ci-avant peut/peuvent être remplacé(s) par D, F, CI, Br, I, CN ou NO₂, ou un système de cycle aromatique ou hétéroaromatique qui comporte 5 à 60 atomes de cycle aromatique, lequel peut dans chaque cas être substitué par un radical ou plusieurs radicaux R⁴, ou un groupe aryloxy ou hétéroaryloxy qui comporte 5 à 60 atomes de cycle aromatique, lequel peut être substitué par un radical ou plusieurs radicaux R⁴, ou un groupe aralkyle ou hétéroaralkyle qui comporte 5 à 60 atomes de cycle aromatique, lequel peut être substitué par un radical ou plusieurs radicaux R⁴, où deux radicaux R³ ou plus peuvent être liés l'un à l'autre ou les uns aux autres et peuvent former un cycle ;
R⁴ est, pour chaque occurrence, de manière identique ou différente, H, D, F ou un radical organique aliphatique, aromatique ou hétéroaromatique qui comporte 1 à 20 atome(s) de C, où, en outre, un ou plusieurs atome(s) de H peut/peuvent être remplacé(s) par D ou F ; deux substituants R⁴ ou plus peuvent ici être liés l'un à l'autre ou les uns aux autres et peuvent former un cycle.

2. Composé selon la revendication 1, **caractérisé en ce que** le groupe de la formule (II) représente un groupe des formules (II-1) à (II-11) qui suivent : 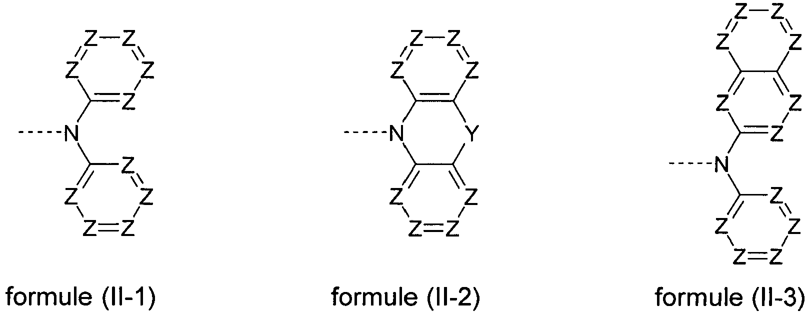 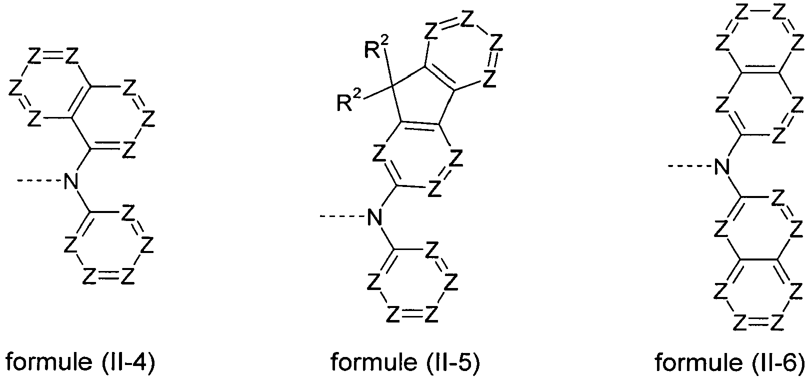 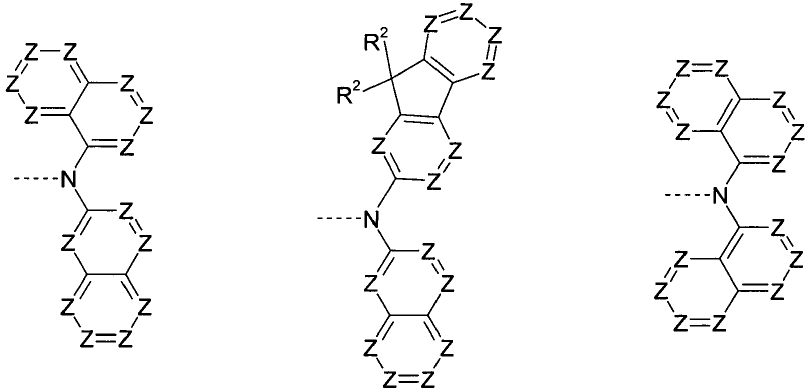 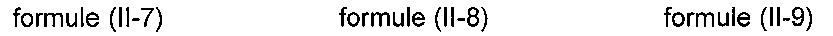 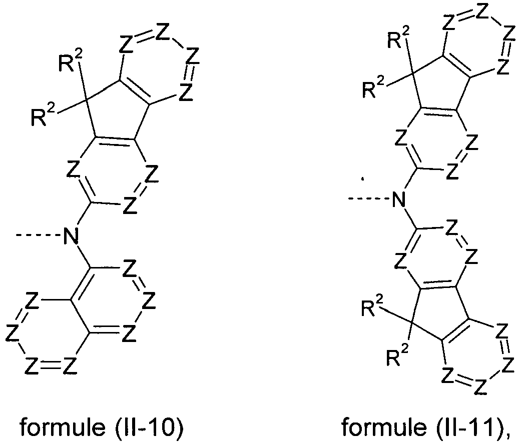 dans lesquelles R² est défini comme selon la revendication 1,
Z représente, pour chaque occurrence, de manière identique ou différente, CR² ou N ; et
Y est choisi parmi une liaison simple, BR², C(R²)₂, Si(R²)₂, NR², PR², P(=O)R², O, S, S=O et S(=O)₂.

3. Composé selon la revendication 1 ou 2, **caractérisé en ce que** les deux groupes Ar¹ qui sont liés à un atome de N commun dans un groupe de la formule (II) sont choisis de manière identique.

4. Composé selon une ou plusieurs des revendications 1 à 3, **caractérisé en ce que** Y est choisi parmi une liaison simple, C(R²)₂, NR², O et S.

5. Composé selon une ou plusieurs des revendications 1 à 4, **caractérisé en ce que** le groupe R¹ est choisi, pour chaque occurrence, de manière identique ou différente, parmi F, CN, Si(R³)₃, ou un groupe alkyle ou alcoxy en chaîne droite qui comporte 1 à 10 atome(s) de C ou un groupe alkyle ou alcoxy ramifié ou cyclique qui comporte 3 à 10 atomes de C, ou un groupe alkényle ou alkynyle qui comporte 2 à 10 atomes de C, où les groupes qui ont été mentionnés ci-avant peuvent chacun être substitués par un radical ou plusieurs radicaux R³ et où un ou plusieurs groupe(s) CH₂ dans les groupes qui ont été mentionnés ci-avant peut/peuvent être remplacé(s) par -C≡C-, -R³C=CR³-, Si(R³)₂, C=O, C=NR³, -NR³-, -O-, -S-, -C(=O)O- ou -C(=O)NR³-, ou un système de cycle aromatique qui comporte 5 à 20 atomes de cycle aromatique, lequel peut dans chaque cas être substitué par un radical ou plusieurs radicaux R³, ou un système de cycle hétéroaromatique qui n'est pas lié via un atome d'azote de cycle, et qui est dans chaque cas substitué par un radical ou plusieurs radicaux R³.

6. Composé selon une ou plusieurs des revendications 1 à 5, **caractérisé en ce que** les radicaux R¹ dans la formule (I) sont choisis de manière identique.

7. Procédé pour la préparation d'un composé selon une ou plusieurs des revendications 1 à 6, **caractérisé en ce qu'**au moins un produit intermédiaire de la formule (Z) : 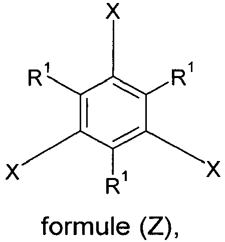 dans laquelle R¹ est défini comme en connexion avec la formule (I) selon la revendication 1, et dans laquelle :
X représente, de manière identique ou différente, n'importe quel groupe réactif souhaité, par exemple CI, Br, I, des groupes d'acide boronique, d'ester d'acide boronique ou d'ester d'acide sulfonique,
est amené à réagir avec au moins un composé arylamino ou au moins un composé hétérocyclique qui contient au moins une fonction NH.

8. Formulation comprenant au moins un composé de la formule (I) selon une ou plusieurs des revendications 1 à 6 et au moins un solvant.

9. Dispositif électronique contenant au moins un composé selon une ou plusieurs des revendications 1 à 6.

10. Dispositif électronique selon la revendication 9, **caractérisé en ce qu'**il est choisi parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

11. Dispositif électroluminescent organique selon la revendication 10, **caractérisé en ce que** le composé selon une ou plusieurs des revendications 1 à 6 est présent en tant que matériau de transport de trous dans une couche de transport de trous, en tant que matériau de matrice dans une couche d'émission, en tant que matériau de blocage d'électrons ou en tant que matériau de blocage d'excitons.

12. Dispositif électroluminescent organique selon la revendication 10 ou 11, **caractérisé en ce que** le composé selon une ou plusieurs des revendications 1 à 6 est présent en tant que matériau de matrice dans une couche d'émission phosphorescente, où, en plus du composé selon une ou plusieurs des revendications 1 à 6, au moins un autre matériau de matrice est présent dans la couche d'émission phosphorescente.
